# EUROPEAN PATENT SPECIFICATION

(11) **EP 2 656 826 B1**
(45) Date of publication and mention of the grant of the patent: **31.10.2018**
(21) Application number: 11851031.2
(22) Date of filing: 14.12.2011
(51) Int. Cl.: A61F 13/49, A61F 13/511, A61F 13/533, A61F 13/537

(54) **ABSORPTIVE ARTICLE**
SAUGFÄHIGER ARTIKEL
ARTICLE ABSORBANT

(30) Priority: 21.12.2010 JP 2010284527; 28.01.2011 JP 2011017053; 31.01.2011 JP 2011018443; 07.12.2011 JP 2011268099
(43) Date of publication of application: 30.10.2013
(73) Proprietor: Kao Corporation, Chuo-Ku Tokyo 103-8210 (JP)
(72) Inventor: YANAGIHARA, Shigeto, Haga-gun Tochigi 321-3497 (JP); TOMITA, Mina, Haga-gun Tochigi 321-3497 (JP); KOUTA, Takuya, Haga-gun Tochigi 321-3497 (JP)
(74) Representative: Vossius & Partner Patentanwälte Rechtsanwälte mbB
(86) International application number: PCT/JP2011/078890
(87) International publication number: WO 2012/086487

(56) References cited:
- WO-A1-01/24755
- WO-A1-01/24756
- WO-A1-2008/072675
- JP-A- 59 207 150
- JP-A- 2000 514 672
- JP-A- 2003 210 507
- JP-A- 2004 174 234
- JP-A- 2005 073 921
- JP-A- 2006 061 396
- JP-A- 2006 115 996
- JP-A- 2006 141 647
- JP-A- 2006 346 210
- JP-A- 2009 172 363
- JP-U- H0 535 126

## Description

### Technical Field

The present invention relates to absorbent articles including a disposable diaper, and the like.

### Background Art

As an absorbent article such as disposable diaper, an article including a liquid-permeable topsheet forming a skin-facing surface, a liquid-impermeable backsheet forming a non-skin-facing surface, and an absorbent member arranged between the two sheets is known. As the absorbent member, an absorbent member including an absorbent core including a hydrophilic fiber such as wood pulp and/or an absorbent polymer and a core wrap sheet which covers the absorbent core is known.

In addition, with respect to the topsheet, a technique in which an uneven structure is provided to the topsheet, whereby flow or diffusion of excrement liquid such as urine or loose stool, in particular comparatively high viscosity excrement liquid such as loose stool in a surface direction is inhibited, is also known. For example, JP 2004-174234 A describes a topsheet having a upper layer facing a skin side of a wearer and a lower layer arranged on an absorbent member side, wherein a large number of junctions are formed by partly joining between the upper layer and the lower layer, and a large number of projections whose insides are cavities are formed by protruding the upper layer toward the skin side of the wearer in parts other than the junctions. Further, JP 2009-136311 A describes that a flow-inhibiting member (for example, a sheet with an uneven surface), which has a lower flowing speed of spurious loose stool than that in other parts of the skin-facing surface, is provided on a part of the skin-facing surface of the topsheet.

With respect to an absorbent core, JP 2006-149571 A describes that in order to improve fit of an absorbent article (absorptive pad) to a body of a wearer, a pair of flexible axes extending in a longitudinal direction of the absorbent core, specifically through holes which pierce the absorbent core in the thickness direction, are formed at a front part in the longitudinal direction of the absorbent core. According to JP 2006-149571 A, due to the presence of a pair of flexible axes which are exclusively located at the front part of the absorbent core put in the crotch of the wearer, even if a pressure is applied to the absorbent core from the lateral direction when it is worn, the front part is not twisted unsuitably, and deforms along with the flexible axis, thus resulting in tight fit to the body of the wearer, and, on the other hand, a rear part does not deform as in the front part and has room, and as a result, the absorbent pad can cover the body of the wearer. The through hole (flexible axis) described in JP 2006-149571 A mainly controls the deformation of the absorbent core when worn, thus resulting in improvement of the fit to the body of the wearer. JP 2006-149571 A, accordingly, does not describe the control of excrement liquid by the through hole.

With respect to a core wrap sheet, JP 2005-566 A describes that a diffusion speed of the core wrap sheet (crepe paper) is made higher than that of the absorbent core (absorptive body). According to JP 2005-566 A, when the diffusion speed is set as described above, the resultant diffusion speed gradient is an incline gradient from the topsheet to the absorbent core, whereby body fluid excreted to the topsheet is not greatly prevented at the intermediate core wrap sheet, and the body fluid can be promptly absorbed in the absorbent core, thus resulting in prevention of leak of the body fluid. JP 2005-566 A describes that the diffusion speed of the crepe paper is set at 0.5 cm³/second or higher, and this diffusion speed can be calculated that a sample for measurement, which is obtained by superimposing 10 sheets to be measured (crepe papers), is fallen to a Petri dish including blood of horse, a time in which the sheet to be measured absorbs the blood of horse (diffusion time) is measured, and the volume of the sample for measurement is divided by the diffusion time.

In order to improve the absorbency or the fit, a technique in which the absorbent core is formed so as to have a part where there is no material forming the absorbent core, or there is the material forming the absorbent core in a comparatively small amount (a channel, through hole, or the like) is known. For example, JP 59-207150 A describes an absorbing structure in which an absorbent core made of cellulose fiber is partly compressed to form a reserve part and channel, whereby the whole amount of urine of adult can be absorbed and kept.

JP 2004-41311 A describes that in order to improve the fit to a body of a wearer, a channel which has a rectangular cross-sectional shape and runs along a longitudinal direction of an absorbent core, is formed on each of a skin-facing surface and a non-skin-facing surface of the absorbent core. According to JP 2009-136311 A, when the absorbent core on which such channels are formed is put on a portion under the crotch of the wearer, it is folded along with the channels, and thus the excellent fit can be provided.

JP 2007-215688 A describes that in order to prevent undesirable twisting such that an absorbent member (absorbent core) rises toward a wearer side when the absorbent article is worn, openings are formed on an absorbent core, the openings piercing the absorbent core in the thickness direction, and continuously or discontinuously extending in the longitudinal direction of the absorbent core.

WO 01/24756 A1 discloses an absorbent article with a central rinsing member and a slit through the whole thickness of the rinsing member. WO 01/24755 A1 discloses an absorbent article with a wicking barrier and a central rinsing member, and a slit in the rinsing member.

### Summary of Invention

Disposable diapers has a problem in which skin grows red or gets a rash by adhering excrement liquid such as urine or stool to a skin of a wearer, when they are worn. Such rough skin of the wearer is often caused by first adhering comparatively low viscosity excrement liquid such as urine to a skin to generate a condition in which the skin swells and is easily damaged, and then adhering stool such as loose stool, which has stronger stimulation to the skin, to the skin in that condition. In order to solve the problem of the rough skin, it is necessary to increase the absorbency to the urine or loose stool and reduce the so-called "wet back" (a phenomenon in which excrement liquid permeating the topsheet is not kept in the absorbent member and returns to the topsheet), whereby the adhesion of the urine or loose stool to the skin is reduced. Patent Literatures 1 to 4 do not particularly describe such a problem of the rough skin of the wearer caused by the urine or stool, and therefore the technique in which the adhesion of the excrement liquid such as urine or loose stool to the skin of the wearer is reduced has not been established yet in the absorbent article.

The present invention relates to providing of an absorbent article having excellent absorbency to excrement liquid such as urine or loose stool, and reduced adhesion of the excrement liquid to the skin of the wearer.

The absorbent article of the present invention is specified in the claims.

A disposable diaper including an absorbent core (absorbent member) has a predetermined thickness and a predetermined rigidity, and therefore, it is not easy to comfortably fit it to each part of a body such as a crotch portion. If the diaper is not fitted comfortably to the body and spaces are generated between the body and the diaper, excrement liquid may sometimes leaks into the outside depending on the degree of the space. Conventional absorbent articles as described in JP59-207150 A, JP2004-41311 A and JP2007-215688 A are insufficiently fitted to the body of the wearer and have a room for improvement.

The present invention also relates to providing of an absorbent article having excellent fit to a body of a wearer, and reduced leakage of excrement liquid.

The present invention provides an absorbent article including an absorbent core, wherein a slit, which does not pierce the absorbent core in the thickness direction, has an opening on a skin-facing surface side or a non-skin-facing surface side of the absorbent core, and extends in a vertical direction of the absorbent article, is formed on the absorbent core, the slit has a part in which the width thereof gradually increases or decreases from the skin-facing surface side to the non-skin-facing surface side of the absorbent core; and the opening of the slit exists on the side at which the slit width is relatively wide.

In absorbent articles, in particular disposable diapers, a technique in which a slit is formed in a longitudinal direction of an absorbent core and both sides of the absorbent core can be easily folded, thereby improving the fit to the portion under the crotch and the leakage prevention, as discussed in JP 3-123553 A, has been hitherto known.

As a technique in which the slit, which pierces or does not pierce the absorbent core of the disposable diaper, is formed, a technique in which the absorbent core is divided along a virtual line extending in a longitudinal direction of a diaper and, at the same time, is divided along a virtual line in a width direction thereof (slits are formed in both of the longitudinal direction and the width direction on the absorbent core) is proposed (JP 11-216161 A). In the diaper in JP 11-216161 A, slits are formed in the width direction so that they intersect a plurality of slits in the longitudinal direction at a portion under the crotch.

In addition, a disposable diaper in which a connecting channel which connects two reserve parts on a vertical axis of a cellulose fiber pad is provided on a surface of the pad is also proposed (JP 59-207150 A).

When the absorbent article such as the diaper is distributed (put on the market, or the like), however, it is often put in a wrapping bag in a state in which it is folded in two or three in the longitudinal direction and compressed, and thus when consumers take out it from the wrapping bag, it has often two or three creases. As a result, even if the slit is formed in the longitudinal direction on the absorbent core, an effect of improving the fit to the crotch, obtained by forming the slit, may be sometimes insufficiently exhibited.

In the diaper in JP 11-216161 A, slits in the width direction intersect a plurality of slits in the longitudinal direction at a portion under the crotch. The diaper is accordingly folded along the slits in the width direction when it is worn, whereby it is difficult to fold the diaper along the slit in the longitudinal direction at intersections with the slit in the width direction. As a result, in the diaper in JP 11-216161 A, an effect of improving the fit, which obtained by forming the slits in the longitudinal direction, is inhibited by the slits in the width direction.

In addition, in the diaper in JP 59-207150 A, only one connecting channel is formed at the central in the width direction of the pad, and thus the pad is folded in two in the width direction when the diaper is worn, thus resulting in deteriorated fit to the crotch.

The present invention further relates to providing of an absorbent article which shows a good effect of improving the fit to the crotch, which is obtained by the slits in the longitudinal direction, even if creases are generated, and provides excellent fit and excellent prevention of leakage.

In a preferred embodiment, the present invention provides an absorbent article including a liquid-permeable topsheet, a liquid-impermeable or liquid-slightly permeable backsheet, and a liquid-retentive absorbent core, which is located between the both sheet, which has a rear portion, a front portion, and a crotch portion, which is located between them, wherein the absorbent article is folded in two or three in a longitudinal direction, a plurality of slits which pierce or do not pierce it, each of which extends in a longitudinal direction of the article, are formed on the absorbent core, and the slits are not connected consecutively to each other at least a portion under a crotch, and have a wide portion having a wide slit width at a position different from the position at which the article is folded in two or three.

### Brief Description of Drawings

[FIG. 1] FIG. 1 is a view showing one example of a disposable diaper which is one embodiment (a first embodiment) of an absorbent article of the present invention, and is a schematic plan view showing a state in which an elastic member in each portion is elongated to expand it into a planar shape from a skin-facing surface side (topsheet side).
[FIG. 2] FIG. 2 is a schematically cross-sectional view showing a cross-section at a line I-I (cross-section in a width direction) in FIG. 1.
[FIG. 3] FIG. 3 is a schematically perspective view showing a part of a topsheet of the disposable diaper shown in FIG. 1, which is enlarged.
[FIG. 4] FIG. 4 (a) and FIG. 4 (b) are each a view showing schematically an arrangement pattern of depressions (junctions) in a topsheet according to the present invention.
[FIG.5] FIG. 5 is an explanatory view of a measurement method of a liquid permeation time.
[FIG. 6] FIG. 6 is a schematically cross-sectional view showing a cross-section in a width direction of another embodiment of an absorbent member according to the present invention.
[FIG. 7] FIG. 7 is a view showing one example of a disposable diaper which is one embodiment (second embodiment) of an absorbent article of the present invention, and is a schematic plan view showing a state in which an elastic member in each portion is elongated to expand it into a planar shape from a skin-facing surface side (topsheet side).
[FIG. 8] FIG. 8 is a schematically cross-sectional view showing a cross-section at a line I-I (cross-section in a width direction) in FIG. 7.
[FIG. 9] FIG. 9 is a schematic view showing a wearing state of the disposable diaper shown in FIG. 7.
[FIG. 10] FIG. 10 (a) and FIG. 10 (b) are each a schematically cross-sectional view showing a slit in a width direction in another embodiment according to the present invention.
[FIG. 11] FIG. 11 is a schematically cross-sectional view showing an absorbent member in a width direction in another embodiment according to the present invention.
[FIG. 12] FIG. 12 is a plan view showing a disposable diaper which is a third embodiment of the present invention.
[FIG. 13] FIG. 13 is a schematic view for explaining a formed position or a size of a slit and its wide portion in the diaper shown in FIG. 12.
[FIG.14] FIG. 14 (a) is a cross-sectional view at a line I-I in FIG. 12, and FIG. 14 (b) is a cross-sectional view at a line II-II in FIG. 12.
[FIG. 15] FIG. 15 is a perspective view showing a state in which the diaper in FIG. 12 is folded in two.
[FIG. 16] FIG. 16 (a) is a perspective view showing a wearing state of the diaper in FIG. 12, and FIG. 16 (b) is a perspective view showing a wearing state of a conventional diaper.
[FIG. 17] FIG. 17 (a) is an outline cross-sectional view showing a cross-section of the diaper in FIG. 12 in a longitudinal direction when the diaper is worn, and FIG. 17(b) is an outline cross-sectional view showing a cross-section of a conventional diaper in a longitudinal direction when the diaper is worn.
[FIG. 18] FIG. 18 (a) is an outline cross-sectional view showing a cross-section of the diaper in FIG. 12 in a width direction when the diaper is worn, and FIG. 18 (b) is an outline cross-sectional view showing a cross-section of a conventional diaper in a width direction when the diaper is worn.
[FIG. 19] FIGS. 19 (a) and 19 (b) are views showing a diaper with a slit piercing the absorbent core in the thickness direction, and FIG. 19 (a) is a view corresponding to FIG. 14 (a), and FIG. 19 (b) is a view corresponding to FIG. 14(b).

### Description

Absorbent articles of the present invention will be explained based on disposable diapers which are preferable embodiments thereof, referring to drawings below.

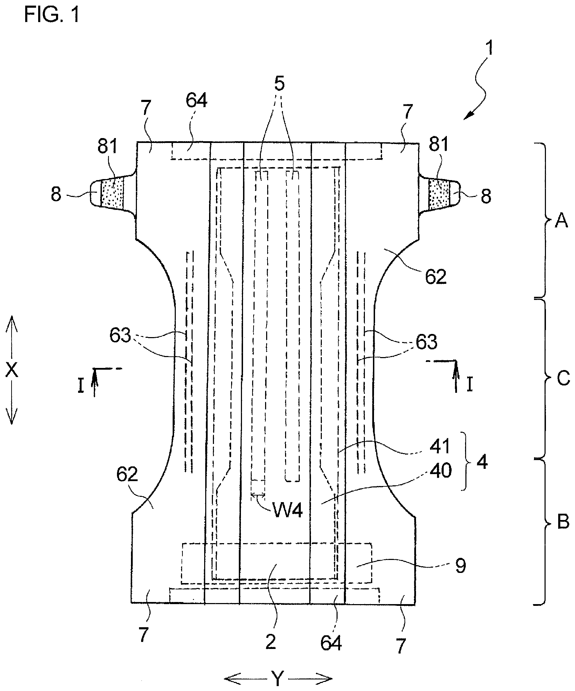

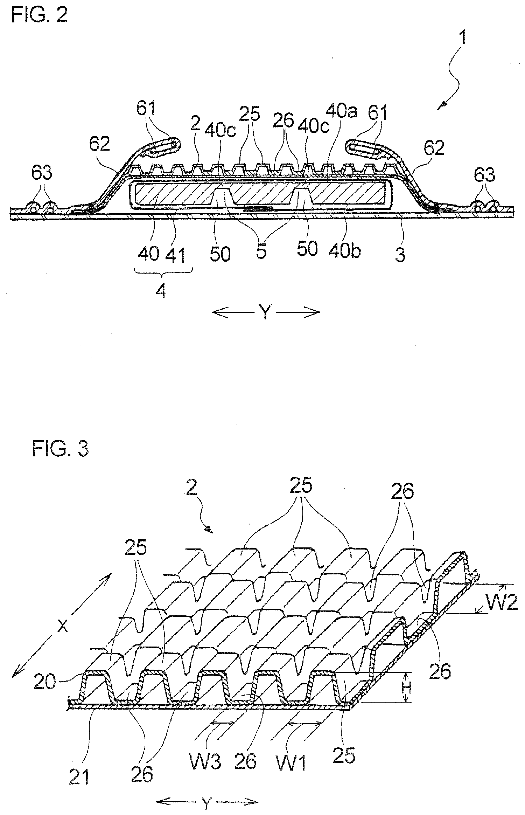

A diaper 1 of a first embodiment, as shown in FIG. 1 and FIG. 2, includes an absorbent member 4 and a topsheet 2 which is arranged on a skin-facing surface side of the absorbent member 4, and the absorbent member 4 includes an absorbent core 40, and a core wrap sheet 41 which covers at least a skin-facing surface 40a of the absorbent core 40.

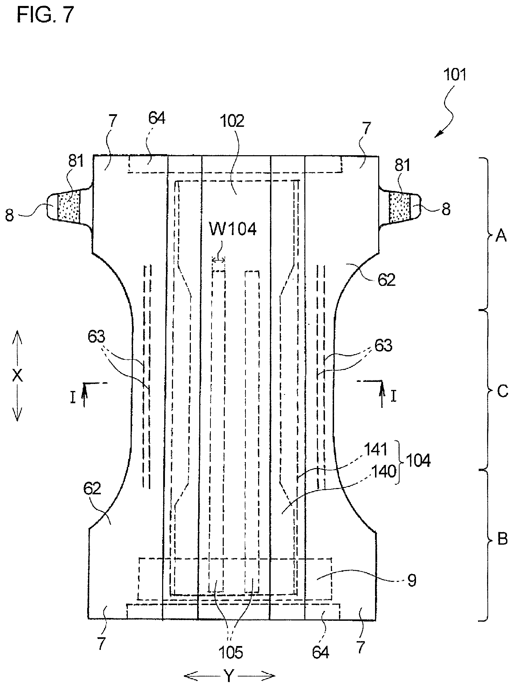

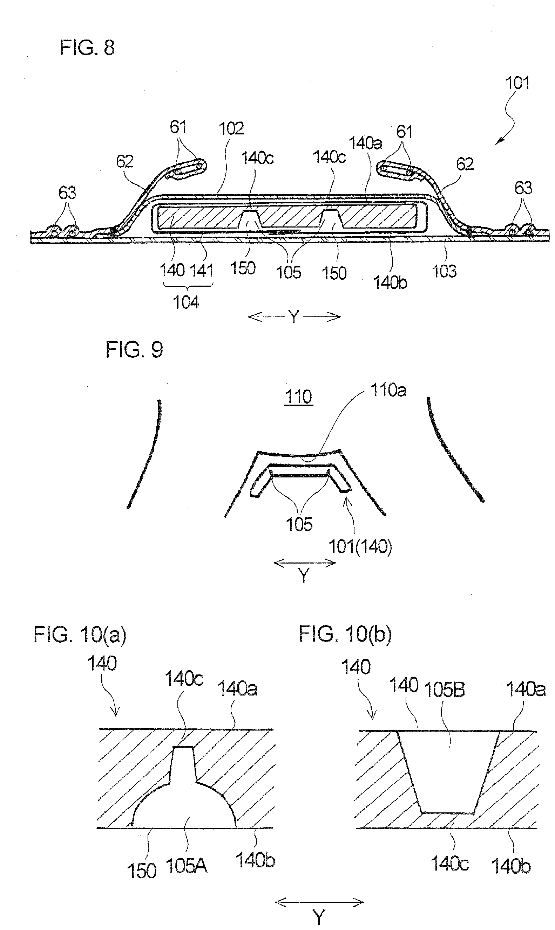

A diaper 101 of a second embodiment, as shown in FIG. 7 and FIG. 8, includes an absorbent core 140 which is long in one direction X. The absorbent core 140 is covered with a core wrap sheet 141 to form an absorbent member 104.

To explain in detail, the diapers 1 and 101 in the first and second embodiments are both a so-called open type disposable diapers, and include, as shown in FIG. 1 and FIG. 2, or FIG. 7 and FIG. 8, a liquid-permeable topsheet 2 or 102 forming a skin-facing surface, a liquid-impermeable or water-repellent (hereinafter correctively referred to as liquid-impermeable) backsheet 3 or 103 forming a non-skin-facing surface, and a liquid-retentive absorbent member 4 or 104 arranged between the both sheets 2 and 3 (or 102 and 103), and are longitudinally formed.

All of the topsheet 2 or 102, the backsheet 3 or 103 and the absorbent member 4 or 104 have a longitudinal shape which is long in one direction (vertical direction) X. The topsheet 2 and the backsheet 3 in the diaper 1 have each a larger size than that of the absorbent member 4, and extend outwardly from a periphery of the absorbent member 4. The topsheet 102 and the backsheet 103 in the diaper 101 have each a larger size than that of the absorbent member 104, and they extend outwardly from a periphery of the absorbent member 104.

The topsheet 2 or 102 has, as shown in FIG. 2 or FIG. 8, a size in the width direction (lateral direction) Y smaller than a size in the width direction (lateral direction) Y of the backsheet 3 or 103.

The diaper 1 or 101 has, as shown in FIG. 1 or FIG. 7, in the longitudinal direction X, a rear portion A which is arranged on a rear side of a wearer when it is worn, a front portion B which is arranged on a front side of the wearer when it is worn, and a crotch portion C which is arranged under a crotch of the wearer when it is worn. The crotch portion C includes a portion facing an excretory part which is arranged so as to face an excretory part of the wearer when it is worn, and is located at a central part in the longitudinal direction X of the diaper 1 or 101. In the diaper 1 or 101, both side edges of the crotch portion C are curved inwardly in the shape of an arc, and the diaper is, in a planar view as shown in FIG. 1 or FIG. 7, sandglass-shaped, in which the central part in the longitudinal direction X is inwardly constricted.

In the present specification, the skin-facing surface refers to a surface which faces a skin side of a wearer in the absorbent article (disposable diaper) or a member forming it (for example, the absorbent core 40 or 140) when the absorbent article is worn, and the non-skin-facing surface refers to a surface which faces an opposite side to the skin side (cloth side) in the absorbent article or the member forming it when the absorbent article is worn. In addition, the longitudinal direction (vertical direction) refers to a direction which is along a long side of the absorbent article or the member forming it, and the width direction (lateral direction) is a direction which is perpendicular to the longitudinal direction. In drawings, a direction shown by reference sign X is a longitudinal direction of the diaper 1 or 101 (absorbent core 40 or 140), and a direction shown by reference sign Y is a width direction of the diaper 1 or 101 (absorbent core 40 or 140).

In the liquid-retentive absorbent core 40 or 140 forming the absorbent member 4 or 104, both side edges of the crotch portion C are curved inwardly in the shape of an arc, and the core is, in a planar view as shown in FIG. 1 or FIG. 7, sandglass-shaped, in which the central part in the longitudinal direction X is inwardly constricted. In addition, the core wrap sheet 41 or 141 forming the absorbent member 4 or 104 is one continuous sheet. As shown in FIG 2 or FIG 8, the sheet covers the whole area of the skin-facing surface 40a or 140a of the absorbent core 40 or 140, and extends outwardly in the width direction Y from both side edge parts along the longitudinal direction X of the absorbent core 40 or 140, the extending parts thereof are rolled below the absorbent core 40 or 140, and the sheet covers the whole area of the non-skin-facing surface 40b or 140b of the absorbent core 40 or 140. The absorbent core 40 or 140 and the core wrap sheet 41 or 141 may be joined in a known joint means such as a hot-melt adhesive, or the like.

As shown in FIG. 1 and FIG. 2, or FIG. 7 and FIG. 8, a side sheet 62, in which threadlike elastic member 61 is fixed in a stretched state on one side edge along the longitudinal direction X, is arranged on each of the both sides of the diaper 1 or 101 along the longitudinal direction X, and a pair of three-dimensional gathers are formed on the crotch portion when the diaper is worn. In addition, threadlike elastic members 63 are arranged in a stretched state along the longitudinal direction X between the topsheet 2 or 102 and the backsheet 3 or 103 on right and left leg portions arranged around legs of a wearer, and a pair of leg gathers are formed by shrinkage of the elastic member 63 on the leg portions when the diaper is worn. Also, a belt-shaped elastic member 64 is arranged in a stretched state along the width direction Y between the topsheet 2 or 102 and the backsheet 3 or 103 on each waist portion (an end in the longitudinal direction X) of the rear portion A and the front portion B, thereby forming a waist gather.

In the diaper 1, a pair of the side sheets 62 and 62, the topsheet 2, the absorbent member 4, the elastic members 63 and 64, and the backsheet 3 are joined to each other with a known joint means such as a hot-melt adhesive. In addition, in the diaper 101, a pair of the side sheets 62 and 62, the topsheet 102, the absorbent member 104, the elastic members 63 and 64, and the backsheet 103 are joined to each other with a known joint means such as a hot-melt adhesive.

As shown in FIG. 1 or FIG. 7, a pair of fastening tapes 8 and 8 are provided on both side edge parts along the longitudinal direction X on the rear portion A of the diaper 1 or 101. More specifically, in the diaper 1 or 101, a pair of side flaps 7 and 7, which outwardly extend from both side edge parts along the longitudinal direction X of the absorbent member 4 to the width direction Y, are formed on the both sides along the longitudinal direction X of the rear portion A and the front portion B, and the fastening tape 8, which outwardly extends in the width direction Y, is attached to each of a pair of the side flaps 7 on the rear portion A. A fixing part 81 made of a male member of a mechanical hook-and-loop fastener is attached to the fastening tape 8.

In addition, a fixed region 9 made of a female member of a mechanical hook-and-loop fastener is formed on the non-skin-facing surface of the front portion B of the diaper 1,101. The fixed region 9 is formed by joining and fixing the female member of the mechanical hook-and-loop fastener on the non-skin-facing surface of the backsheet 3 with a known joint means (for example, using an adhesive, heat-sealing, or the like), and the fixing part 81 can be attachably and detachably fixed on the fastening tape 8.

In the diaper 1 of the first embodiment, the absorbency to the excrement liquid such as urine or loose stool is increased, thus resulting in reduced adhesion of the excrement liquid to the skin of the wearer. The topsheet 2, the absorbent core 40 and the core wrap sheet 41 are each devised to exert such an effect, and the devisal is a main feature of the present embodiment. The main feature parts of the diaper 1 of the first embodiment will be explained below.

The topsheet 2 of the first embodiment has, as shown in FIG. 2 and FIG. 3, a large number of projections 25 protruding toward the skin of a wearer. A large number of depressions 26 are formed on the skin-facing surface of the topsheet 2 in addition to a large number of the projections 25, and thus an uneven shape including a large number of projections 25 and depressions 26 is formed. The uneven shape of the skin-facing surface of the topsheet 2 is, as shown in FIG. 2, formed at least above the absorbent core 40 in the crotch portion C (the portion facing the excretory part). In the present embodiment, the uneven shape is further formed above the absorbent core 40 in each of the rear portion A and the front portion B. On the other hand, the non-skin-facing surface (the surface facing the absorbent member 4) of the topsheet 2 is a flat surface with no projections and depressions.

A topsheet 102 in a second embodiment and topsheets in other embodiments described below may have the structure as that of the topsheet 2 in the first embodiment.

The topsheet 2 will be explained in more detail. The topsheet 2 has, as shown in FIG. 3, an upper layer 20 facing the skin side of the wearer, and a lower layer 21 arranged on the absorbent member 4 side. The upper layer 20 and the lower layer 21 are partially joined to each other on the bottom of each of a large number of the depressions 26, and the bottom of each depression 26 serves as a junction between the upper layer 20 and the lower layer 21. The upper layer 20 protrudes toward the wearer skin in parts other than the bottom of the depression 26 (junctions) to form the projection 25. A space is formed inside the projections 25. The bottom of the projections 25 is rectangular. The projection 25 is wholly a flat rectangular parallelepiped or truncated quadrangular pyramid with a rounded ridge line. On the other hand, the depression 26 (junction) is also rectangular.

The projections 25 and the depressions 26 (junctions) are arranged alternatively so that they form a line in one direction. In the present embodiment, as shown in FIG. 3, the projections 25 and the depression 26 are alternatively arrange to form a line along the width direction Y of the diaper 1. The plurality of lines formed of the projections 25 and the depressions 26, which extend in the width direction Y, are arranged serially in the longitudinal direction X of the diaper 1.

With respect to the topsheet 2, in a case in which any one of the projections 25 in one line extending in the width direction Y of the diaper 1 is focused, there is no projection 25 at a position adjacent to the above-mentioned projection 25 in another line adjacent to this line. The phrase "there is no projection at a position adjacent to one projection" means that when one projection 25 is focused, there is no projection 25 at the completely same position in a line adjacent to this line. In other words, it means that with respect to the longitudinal direction X of the diaper 1 in FIG. 3, the projections 25 are arranged so that the projections 25 in lines adjacent to each other do not completely lie in a row. At a position adjacent to one projection 25, accordingly, there may be both of a part of a projection 25 and a part of a depression 26 (junction), or there may be only a depression 26. In the present embodiment, the depressions 26 are arranged so that they are staggered a half pitch in two lines of projections 25 adjacent to each other. When any one of the depressions 26 in one line is focused, accordingly, the depression 26 is formed so that its front and back and right and left are surrounded by projections 25 to form a closed depression. The depressions 26 (junctions) are, therefore, arranged in a hound's-tooth check pattern, and accordingly the projections 25 are arranged in the same hound's-tooth check pattern.

As described above, when the uneven shape formed of a large number of projections 25 and depressions 26 (junctions) is formed on the skin-facing surface of the topsheet 2, because the excrement liquid, in particular the loose stool, which is the high viscosity excrement liquid is trapped in the depression 26, it is difficult to diffuse it in a surface direction, and thus side flowing of the loose stool can be inhibited. In addition, because the excrement liquid such as the loose stool is trapped in the depression 26, downward transfer of the excrement liquid (that is, a direction to an absorbent member) is promoted, thus resulting in prevention of leakage of the excrement liquid. In particular, in the present embodiment, due to the spaces formed in the projections 25, as a transfer path of the excrement liquid trapped in the depressions 26 into the absorbent member 4, there are paths from the sides of the projections 25 surrounding the depression 26 to the downward direction through spaces inside the projections 25, in addition to paths from the bottoms (junctions) of the depression 26 to a downward direction (a direction to the absorbent member), and thus pulling property of the excrement liquid such as the loose stool is excellent, and a chance to contact the excrement liquid with skin is reduced.

In terms of the more reliable exertion of the effect obtained by such an uneven shape of the topsheet 2, it is preferable to set a size and the like of the projections 25 as follows:
The height H (see FIG. 3) of the projection 25 is preferably from 1 to 10 mm, more preferably from 3 to 6 mm.

The bottom size W1 of the projection 25 along the width direction Y (see FIG. 3) on the diaper 1 is preferably from 2 to 30 mm, more preferably from 2 to 5 mm; and the bottom size W2 along the along the longitudinal direction X (a direction perpendicular to the direction of the lines of the projections 25 and the depressions 26) (see FIG. 3) on the diaper 1 is preferably from 2 to 30 mm, more preferably from 2 to 5 mm.

The base area of the projection 25 is preferably from 4 to 900 mm², more preferably from 4 to 25 mm².

The length W3 of the depression 26 (junction) in the width direction Y (a direction of the lines of projections 25 and the depressions 26) on the diaper 1 (see FIG. 3) is preferably from 0.1 to 20 mm, more preferably from 0.5 to 5 mm.

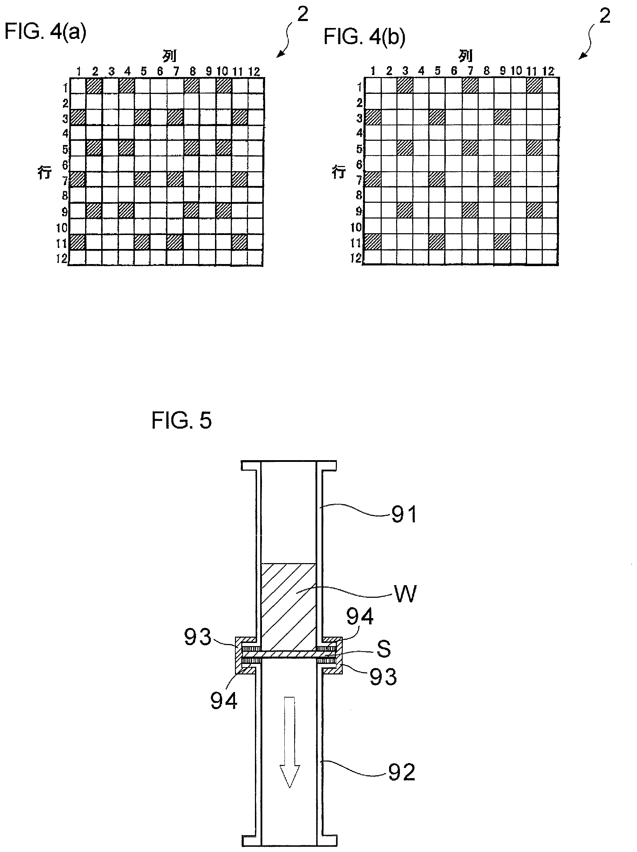

As described above, the projections 25 and the depressions 26 (junctions) are arranged in a hound's-tooth check pattern on the topsheet 2, and the hound's-tooth check pattern of these projections 25 (depressions 26) can be set, for example, as shown in FIG. 4. FIG. 4 (a) shows a pattern in which one projection 25 is surrounded by 6 depressions 26 (a 6-depression pattern), and FIG. 4 (b) shows a pattern in which one projection 25 is surrounded by 4 depressions 26 (a 4-depression pattern). Both of the patterns are a pattern in which the projections 25 are arranged in the hound's-tooth check pattern. In FIG. 4, the positions of the depression 26 with 12 rows and 12 columns (the projections 25 are not shown) are shown, and shaded squares shown in FIG. 4 are the depressions 26.

In a 6-depression pattern shown in FIG. 4 (a), a first 6-point pattern element (a first element) is configured with 6 depressions (1, 2), (1, 4), (3, 1), (3, 5), (5, 2) and (5, 4). Here, the numbers in parentheses () show (the row number, the column number). A second element in the next to the right is configured with 6 depressions (1, 8), (1, 10), (3, 7), (3, 11), (5, 8) and (5, 10). Further, a third element below the first element is configured with the total 6 depressions of depressions (7, 1), (7, 5), (9, 2) and (9, 4), and the two depressions (5, 2) and (5, 4), which are in the lower part of the first element and are shared. A fourth element in the next to the right is configured with the total 6 depressions of the (3, 5), (3, 7), (5, 4), (7, 5) and (5, 8), which are shared with any of the first to third elements, and a depression (7, 7). As described above, the skin-facing surface of the topsheet 2 shown in FIG. 4 (a) is configured so that the 6-depression pattern is widely repeated while the depressions 26 in a part of each element are shared with each other.

In a 4-depression pattern shown in FIG. 4 (b), a first element is configured with 4 depressions (1, 3), (3, 1), (3, 5) and (5, 3). A second element in the next to the right is configured with depressions (1, 7), (3, 9) and (5, 7), and the depression (3, 5) which is shared. A third element shown below the first element is configured with depressions (7, 1), (7, 5) and (9, 3), and the depression (5, 3) which is shared with the first element. A fourth element in the next to the right is configured with the depressions (3, 5), (5, 3), (5, 7) and (7, 5) which are shared with any of the first to third elements. As described above, the skin-facing surface of the topsheet 2 shown in FIG. 4 (b) is configured so that the 4-depression pattern is widely repeated while the depressions 26 in a part of each element are shared with each other.

In the arrangement patterns of the depressions 26 (junctions) shown in FIG. 4(a) and FIG. 4 (b), it is preferable that the projection 25 (an apex part of the projection 25) is located roughly in the center of each element configured with the 6 or 4 depressions 26. For example, in a case of the first to fourth elements of 6-depression pattern in FIG. 4 (a), it is preferable that the apex part of the projection 25 is put on each of (3, 3) in the first element,(3, 9) in the second element, (7, 3) in the third element, and (5, 6) in the fourth element. In a case of the first to fourth elements of 4-depression patter in FIG. 4 (b), it is preferably that the apex part of the projections 25 is put on each of (3, 3) in the first element, (3,7) in the second element, (7,3) in the third element, and (5,5) in the fourth element. As described above, when the projections 25 are arranged in a hound's-tooth check pattern, a pressure applied to the skin of the wearer is uniformly dispersed, and more excellent cushioning property is realized. In addition, preferably the absorbing and keeping property of the excrement liquid can be uniformly exhibited throughout the topsheet.

The upper layer 20 and the lower layer 21 which form the topsheet 2 are made of the same or different sheet of a fiber material. This sheet is substantially non-elastic. The substantially non-elastic sheet refers to a sheet in which its elongation limit is, for example, 105% or less, and material failure occurs or permanent stress is generated at a value over the elongation limit. As such a sheet, any material used as the topsheet in conventionally known absorbent articles, which is substantially non-elastic, can be used without any limitation. The sheet may include, for example, various non-woven fabrics such as a non-woven fabric produced in a card method, a spunbonded non-woven fabric, a molt-blown non-woven fabric, a spun lace non-woven fabric and a needle punched non-woven fabric; films capable of permeating liquid by an opening means, and the like. When the non-woven fabric is used as the sheet for the upper layer 20 and the lower layer 21, it is preferable that the fineness of the forming fiber is from 1 to 20 dtex, particularly from 1.5 to 4 dtex, in terms of the secure of the strength of the topsheet and the improvement of the texture. It is preferable to subject both the upper layer 20 and the lower layer 21 to a hydrophilization treatment using a surfactant, and the like.

The upper layer 20 has, preferably, a basis weight of 10 to 100 g/m², particularly 10 to 30 g/m². On the other hand, the lower layer 21 has, preferably, a basis weight of 5 to 50 g/m², particularly 10 to 30 g/m². The topsheet 2 including the upper layer 20 and the lower layer 21 has, preferably, a total basis weight of 15 to 150 g/m², particularly 20 to 60 g/m².

The topsheet 2 having such an uneven shape can be produced utilizing a known emboss processing. For example, an original fabric of the upper layer 20 is supplied to an engaged part between a first roll whose peripheral surface has an uneven shape and a second roll whose peripheral surface has an uneven shape and is engaged with the uneven shape of the first roll to give the unevenness to the original fabric of the upper layer 20, and then the resulting uneven original fabric of the upper layer 20 is put on an original fabric of the lower layer 21 to obtain a laminate. The resulting laminate is heated and pressed to join the two original fabrics with heat-seal, whereby the topsheet 2 having the uneven shape as shown in FIG. 3 and FIG. 4 can be obtained. The content described in JP 2004-174234 A, which is the previous application filed by the same applicants, can be suitably applied to the topsheet 2 according to the present embodiment.

In the first embodiment, the absorbent core 40 is further devised in addition to the devisal of the topsheet 2 described above. Specifically, on the absorbent core 40 in the present embodiment, as shown in FIG. 1 and FIG. 2, slits 5, which have an opening 50 at least on the non-skin-facing surface 40b side of the absorbent core 40 and extend in the longitudinal direction X of the absorbent core 40, are formed. On the absorbent core 40, a plurality of (two) slits 5, which extend in the longitudinal direction X and are continuous straight line shaped (a rectangular shape), are formed in the width direction Y of the absorbent core 40 at a predetermined interval. These two slits 5 and 5 are parallel to each other and have the same length in the longitudinal direction X, and are symmetrically formed across a virtual straight line (not shown) which divides the absorbent core 40 in two in the width direction Y. The interval between the two slits 5 and 5 is preferably from 10 to 50 mm.

With respect to the diaper 101 of the second embodiment, one of the main features is that slits 105 having a specific shape are formed on the absorbent core 140. Specifically, on the absorbent core 140 in the second embodiment, as shown in FIG. 7 and FIG. 8, slits 105, which do not pierce the absorbent core 140 in the thickness direction, have an opening 150 on the non-skin-facing surface 140b side of the absorbent core 140, and extend in the longitudinal direction X of the absorbent core 140 are formed. On the absorbent core 140, a plurality of (two) slits 105, which extend in the longitudinal direction X and are continuous straight line shaped (a rectangular shape), are formed in the width direction Y of the absorbent core 140 at a predetermined interval. These two slits 105 and 105 are parallel to each other and have the same length in the longitudinal direction X, and are symmetrically formed across a virtual straight line (not shown) which divides the absorbent core 140 in two in the width direction Y. The interval between the two slits 105 and 105 is preferably from 10 to 50 mm.

The slit 105 on the diaper 101 has, as shown in FIG. 8, a portion in which the width W 104 (see FIG. 7, a length in the direction Y perpendicular to the extending direction X of the slit 105) is gradually increased from the skin-facing surface 140a side to the non-skin-facing surface 140b side of the absorbent core 140, and there is the opening 150 at an end in the thickness direction (the depth direction of the slit 105) of the side at which the width W 104 is relatively wide. The slit 105 in the second embodiment has, as shown in FIG. 8, a shape of (isosceles) trapezoid in a section view in the width direction Y, and the width W104 is gradually increased from the skin-facing surface 140a side to the non-skin-facing surface 140b side in the whole area of the slit 105. When the width W104 of the slit 105 is gradually increased from the skin-facing surface 140a side to the non-skin-facing surface 140b side as described above, the absorbent core 140 (diaper 101) is more easily bent to a side at which the width W104 is relatively wider (non-skin-facing surface 140b side) at the slit 105 as a line of bending, compared to a case in which, for example, the width W104 is not changed in the thickness direction of the absorbent core 140 (for example, in a case in which the slit 105 has a rectangular cross-sectional shape), as described below (see FIG. 9).

The slit 5 or 105 is formed at least on the crotch portion C (the portion facing the excretory part described above).

The slit 5 in the first embodiment, as shown in FIG 1, continuously and linearly extends from the vicinity of the end of the absorbent core 40 on the rear portion A side in the longitudinal direction to a portion in the front portion B near to the crotch portion C through the crotch portion C (the portion facing the excretory part described above). The length in the front portion B is shorter than those in the rear portion A and the crotch portion C, and thus the slit deviates to the rear portion A side, including the crotch portion C. The formation position of the slit 5 in the first embodiment corresponds to the area in which loose stool is excreted and its vicinity.

The slit 105 in the second embodiment, as shown in FIG. 7, continuously and linearly extends from the vicinity of the end of the absorbent core 140 on the front portion B side in the longitudinal direction to a portion in rear portion A near to the crotch portion C through the crotch portion C (the portion facing the excretory part described above). The length in the rear portion A is shorter than those in the front portion B and the crotch portion C, and thus the slit deviates to the front portion B side, including the crotch portion C. The formation position of the slit 5 in the second embodiment corresponds to the area in which urine is excreted and its vicinity.

The slit 5 or 105 is a portion which is formed by intentionally inhibiting the accumulation of materials forming the absorbent core 40 or 140, in the accumulation step of the materials forming the absorbent core 40 or 140 (for example, fiber materials such as wood pulp, particle of an absorbent polymer, and the like) in the production of the absorbent core 40 or 140, and there are substantially no materials forming the absorbent core 40 or 140 in the slit 5 or 105. The phrase "there are substantially no forming materials in the slit" includes a case in which there are unintentionally a slight amount (a basis weight lower than the basis weight of a peripheral part of the slit 5 or 105 in the absorbent core 40 or 140) of the materials forming the absorbent core 40 in the slit 5 or 105.

The absorbent core 40 or 140 having the slit 5 or 105 can be produced according to a production method of a conventionally known absorbent core. For example, in a method in which materials forming the absorbent core 40 or 140, which is supplied by air flow, are sucked into a forming mold formed on a circumference surface of a rotating drum, and are accumulated to obtain the absorbent core 40 or 140, the forming mold having a predetermined pattern (for example, a forming mold in which a portion corresponding to the slit 5 or 105 protrudes more upwardly compared to a peripheral part) is used as the forming mold, whereby the above-mentioned core can be obtained. In the thus obtained absorbent core 40 or 140, a portion in which the forming materials are not accumulated (a portion in which there are substantially no forming materials) is the slit 5 or105.

The slit 5 or 105 in the first or second embodiment, as shown in FIG. 2 or FIG. 8, does not pierce the absorbent core 40 or 140 in the thickness direction, and the materials forming the absorbent core 40 or 140 are accumulated between the slit 5 or 105, and a portion at which the core wrap sheet 41 or141 covers the skin-facing surface 40a or 140a of the absorbent core 40 or 140 [i.e., a portion at which the absorbent core 40 or 140 is adjacent to the slit 5 or 105 in the thickness direction of the absorbent core 40 or 140(a side opposite to the opening 50 or 150)], whereby a portion facing the opening 40c or 140c is formed. The portion facing the opening 40c or 140c is not compressed in the thickness direction during the production of the absorbent core 40 or 140, as the other portions except for the slit 5 or 105 in the absorbent core 40 or 140, and therefore is not consolidated. The portions which are not consolidated in the absorbent core 140 are the slit 105, and the portion facing the opening 140c, which is located at the side opposite to the opening 150 of the slit 105.

The portion facing the opening 40c or 140c, which is not consolidated, can be obtained by the production method of the absorbent core 40 or 140 described above, in which the materials forming the absorbent core 40 or 140 are not intentionally accumulated but the slit 5 or 105 is formed. If methods other than the above-mentioned method in which the slit is formed, for example, a method in which an absorbent core having no slit is produced, and a predetermined portion on the resulting absorbent core is subjected to a post-processing such as emboss processing, die cutting or cutting to form a slit are used, the portion facing the opening 40c or 140c, which is not consolidated, cannot be obtained. This is because, if the post-processing is conducted, the absorbent core is considerably compressed in the thickness direction; as a result, the portion facing the opening, which is adjacent to the slit obtained through the post-processing in the thickness direction, is consolidated by the post-processing.

The portion facing the opening 40c, which is not consolidated, has usually a density which is the same as or lower than the density of the peripheral part of the portion facing the opening 40c on the absorbent core 40 (an area within 5mm from the portion facing the opening 40c on the absorbent core 40 except for the slit 5). The reason why the portion facing the opening 40c, which is not consolidated, has the density lower than that of the peripheral part is as follows. In a carrying step after the slit 5 (the portion facing the opening 40c) is formed during the production of the absorbent core 40, when a pressure is applied to the whole absorbent core 40 in the thickness direction by various rolls such as carrying rolls, the peripheral part of the portion facing the opening 40c is compressed to some extent by the pressure, and thus the density may be sometimes increased, but little pressure is applied to the portion facing the opening 40c, and thus it is not substantially compressed, because a real thickness (an accumulation thickness of the materials forming the absorbent core 40) thereof is smaller than that of the peripheral part thereof, and the portion facing the opening is thin. In other words, during the production of the absorbent core 40, the density of the portion facing the opening 40c is not substantially changed, but the density of the peripheral part thereof may be sometimes increased due to the compression while it is carried. In such a case, the density of the portion facing the opening 40c becomes relatively lower.

When the slit 5, which has substantially no materials forming the absorbent core 40 and extends in the longitudinal direction X of the absorbent core 40, is formed on the absorbent core 40, the excrement liquid such as loose stool, which is locally excreted into the portion facing the excretory part of the crotch portion C, promptly diffuses in the longitudinal direction X of the absorbent core 40 through the slit 5 as a conduction path, and thus an area at which the excrement liquid is substantially absorbed on the absorbent core 40 is increased, whereby the wet back of urine or loose stool can be reduced. In particular, in the excretion at an early stage, the slit 5 functions as a reserve part for temporarily storing the excrement liquid at the early state, and thus the absorbency to the excrement liquid such as loose stool is improved.

In addition, in the first embodiment, as described above, the slit 5 does not pierce the absorbent core 40 in the thickness direction, and the portion facing the opening 40c, which is formed by accumulating the materials forming the absorbent core 40 and is not consolidated, is formed on the skin-facing surface 40a side of the portion at which the slit 5 is formed on the absorbent core 40, and thus the excrement liquid, which reaches the skin-facing surface 40a of the absorbent core 40 after permeating through the topsheet 2 and the core wrap sheet 41 in order, is diffused at the portion facing the opening 40c in the width direction Y, whereby it is possible to utilize the absorbent core 40 as a whole for absorbing and keeping the excrement liquid, and the effect of reducing the wet back of urine or loose stool can be more improved.

If there is no portion facing the opening 40c, in other words, if the slit 5 pierces the absorbent core 40 in the thickness direction, the pierced slit 5 divides the diffusion of the excrement liquid in the width direction Y, and thus the improvement effect of utilization efficiency of the absorbent core 40 cannot be really expected. Even if there is the portion facing the opening 40c, when it is consolidated [a case in which the portion facing the opening 40c has a density higher than that of other portions of the absorbent core 40 except for the slit 5 (for example, the peripheral part of the portion facing the opening 40c)], the excrement liquid flows out at the skin-facing surface (a surface facing the topsheet 2) of the absorbent member 4 and it is difficult to enter the absorbent member 4, and thus the diffusion effect of the excrement liquid in the width direction Y in the absorbent member 4 can be insufficiently obtained.

The slit 5 has a portion in which the width W4 (see FIG. 1, a length in the direction Y perpendicular to the extending direction X of the slit 5) is gradually increased from the skin-facing surface 40a side to the non-skin-facing surface 40b side of the absorbent core 40. The slit 5 in the present embodiment has, as shown in FIG. 2, a shape of (isosceles) trapezoid in a section view in the width direction Y, and the width W4 is gradually increased from the skin-facing surface 40a side to the non-skin-facing surface 40b side in the whole area of the slit 5. When the width W4 of the slit 5 is gradually increased from the skin-facing surface 40a side to the non-skin-facing surface 40b side as described above, an effect in which the liquid diffusion by the absorbent member 4 (absorbent core 40) on the non-skin-facing surface side becomes better can be exerted, compared to a case in which, for example, the width W4 is not changed in the thickness direction of the absorbent core 40 (for example, in a case in which the slit 5 has a rectangular cross-sectional shape).

In terms of the more reliable exertion of the effects obtained by the slit 5, as described above, slit 5 has a width W4 (see FIG. 1) of preferably 1 to 20 mm, more preferably 1 to 10 mm. The width W4s, which is the narrowest in the width W4 of the slit 5, is preferably from 1 to 10 mm, more preferably from 1 to 5 mm. From the same viewpoint, a ratio of a total W50 of the narrowest widths W4s of all slits 5 on the crotch portion C (the portion facing the excretory part described above) to a total width W0 in the width direction Y of the absorbent core 40 on the crotch portion C (W50/W0) is preferably from 0.02 to 0.3, more preferably from 0.05 to 0.25.

In addition, in the first embodiment, the core wrap sheet 41 is also devised in addition to the devisal to the topsheet 2 and the absorbent core 40 described above. The portion which covers the skin-facing surface 40a of the absorbent core 40 on the core wrap sheet 41 (the portion arranged between the topsheet 2 and the absorbent core 40) has a liquid permeation time of low viscosity liquid (physiological saline), as measured by the following method, of 2.5 seconds or shorter, preferably 0.5 to 2.5 seconds, even more preferably 0.5 to 2 seconds, particularly preferably 0.5 to 1.5 seconds. The core wrap sheet 41 in the present embodiment has the same structure as a whole, and the liquid permeation time (including a liquid permeation time of high viscosity liquid described below) in an arbitrary portion is within a specific area. The core wrap sheet 41 having the liquid permeation time within the range described above is excellent in liquid-permeability of, in particular, comparatively low viscosity excrement liquid such as urine, and can make the urine quickly permeate into the absorbent core 40 side.

The core wrap sheet 141 in the second embodiment and core wrap sheets, which cover the skin-facing surface of the absorbent core, in other embodiment described below may have the same structure as of core wrap sheet 41 in the first embodiment.

### <Measurement Method of Liquid Permeation Time of Low Viscosity Liquid>

As shown in FIG. 5, two cylinders 91 and 92 having an inside diameter of 35 mm, whose upper and lower ends are open, are arranged up and down bringing axises of the both cylinders 91 and 92 in line, a 8 square cm sheet S to be measured (core wrap sheet) is put between the upper and bottom cylinders 91 and 92. At this time, it is preferable that a clip 93 is fitted in a ring flange provided at the lower end of the upper cylinder 91 and the upper end of the lower cylinder 92 to link the upper and lower cylinders 91 and 92 to each other. The reference sign 94 shows a rubber packing having a through hole with the same size and the same shape as the inside diameter of the cylinders 91 and 92. While the conditions in which the sheet to be measured S is put between the upper and lower cylinders 91 and 92 and fixed, 40 g ± 1 g of physiological saline (The Japanese Pharmacopoeia physical saline OTSUKA NORMAL SALINE is used), which is shown as a reference symbol W in FIG. 5, is supplied into the upper cylinder 91. The physiological saline is supplied into the upper cylinder 91 by pouring the physiological saline from a container (such as a beaker) including the 40 g ± 1 g of physiological saline, which is not shown, at once without pausing (the flow of the physiological saline from the container into the cylinder 91 is not interrupted, and continued to the end) into the upper cylinder 91. The physiological saline is acclimated in a thermostatic chamber at 23°C and 50% RH over one day before use. The physiological saline supplied permeates through the sheet S to be measured or is absorbed into the sheet to be measured S, thereby disappearing it from the upper cylinder 91. A time until a liquid surface of the physiological saline reaches a position of a surface of the sheet to be measured S (a surface on the upper cylinder 91 side) from the supply starting time of the physiological saline is measured, and the time is defined as a liquid permeation time of the low viscosity liquid.

In addition to that a portion which covers the skin-facing surface 40a of the absorbent core 40 in the core wrap sheet 41 (a portion arranged between the topsheet 2 and the absorbent core 40) has a liquid permeation time of a low viscosity liquid of 2 seconds or shorter, it has a liquid permeation time of high viscosity liquid (mixed liquid of glycerol and deionized water), as measured by the following method, of 500seconds or shorter, preferably 50 to 300 seconds, more preferably 50 to 200 seconds. The core wrap sheet 41 having the liquid permeation time within the range described above is excellent in liquid-permeability of, in particular, comparatively high viscosity excrement liquid such as loose stool, and can make the loose stool quickly permeate into the absorbent core 40 side.

### <Measurement Method of Liquid Permeation Time of High Viscosity Liquid>

The measurement is performed in accordance with <Measurement Method of Liquid Permeation Time of Low Viscosity Liquid> described above, except that 10 g ± 1 g of high viscosity liquid having a viscosity of 290 mPa·s is used instead of 40 g ± 1 g of physiological saline. Using a digital pump (a trademark "Masterflex" manufactured by Cole-Parmer Instrument Company), 10 g ± 1 g of high viscosity liquid is supplied to the upper cylinder 91 at a speed of 100 g/ml, and a time until a liquid surface of the high viscosity liquid reaches a position of a surface S of the sheet to be measured (a surface on the upper cylinder 91 side) from the supply starting time of the high viscosity liquid is measured, and the time is defined as a liquid permeation time of the high viscosity liquid. The high viscosity liquid is prepared as follows. Specifically, glycerol and deionized water are mixed in a mass ratio of the former : the latter = 94 : 6, to which 0.01 g, based on the 100 of the mixed liquid of Red No. 2 is added, thereby preparing the desired high viscosity liquid. The high viscosity liquid is acclimated in a thermostatic chamber at 23°C and 50% RH over one day before use.

One example of the core wrap sheet 41 having the liquid permeation times in the low viscosity liquid and the high viscosity liquid within the ranges described above is exemplified by a tissue paper, which is mainly made from bleached Kraft pulp of a coniferous tree having a freeness of 400 to 550 ml, to which a paper strong agent is added, and which has a basis weight of 10 to 14.5 g/m² and a density of 0.05 to 0.2 g/cm³. This tissue paper will be explained.

The tissue paper (core wrap sheet 41) includes, as an essential component, bleached Kraft pulp (NBKP) of a coniferous tree having a freeness of 400 to 550 ml. When the tissue paper includes two or more NBKPs, it is enough that the mixture of the two or more NBKPs has a freeness of 400 to 550 ml. The freeness is a value shown by Canadian standard freeness (C. S. F.) in accordance with JIS P 8121, and a value showing a degree of beating (a treatment in which a pulp is mechanically beaten and ground in the presence of water) of a pulp. In usual, the smaller of the freeness value, the stronger the degree of the beating, the damage of fiber by the beating is great, and the fibrillation is progressive. In the NBKP having the freeness within the range described above, the fibers are easily intertwined each other due to the progressive fibrillation, and therefore, even if the number of nods between the fibers in the configuration fiber is decreased by making the basis weight of the tissue paper lower (making the density lower) in terms of the improvement of the liquid-permeability, the strength of such an NBKP in the bonding between the fibers is higher than that of an NBKP having a freeness of more than 550 ml, in which the fibrillation is not relatively progressive. The tissue paper including mainly the NBKP with the freeness of 400 to 550 ml can, accordingly, have a good strength property.

The NBKP used in the present invention has a freeness of preferably 475 to 525 ml, more preferably 490 to 510 ml. When the freeness is less than 400 ml, the effect of improving the strength, obtained by intertwining the fibers, is saturated, and the cutting of the fibers is promoted, and thus the permeating time may be longer. The beating of NBKP can be carried out in accordance with a usual method using a paper stock (slurry) in which the NBKP is dispersed and using a known beating machine such as a beater or a disk refiner. As the NBKP used in the present invention, NBKP, which is usually used in this kind of paper, can be used without particular limitations. As the NBKP, ECF (elemental·chlorine free) bleached pulp in which a chlorine compound is not used for breaching the pulp, or TCF (total·chlorine free) bleached pulp may be used.

The tissue paper includes mainly NBKP having a freeness of 400 to 550 ml. Here, the phrase "include mainly" means that a content of the NBKP having the freeness within the range described above is 50% by mass or more. The content is preferably from 50 to 100% by mass, more preferably from 80 to 100% by mass, in terms of achievement of good strength property.

The tissue paper may include another fiber except for NBKP, and the other fibers may be fibers different from hydrophilic cellulose fibers such as NBKP. The other fibers may include, for example, broad-leaved tree bleached Kraft pulp (LBKP); coniferous tree bleached sulfite pulp (NBSP); wood pulp such as thermomechanical pulp (TMP); a bast fiber such as paper mulberry, paper birch or ganpi (Diplomorpha sikokiana)); non-wood pulp such as straw, bamboo, kenaf, or hemp; synthetic fibers such as a polyester fiber, a rayon fiber, and an acrylic fiber, and the like. These other fibers may be preferably included in a content of 50% by mass or less.

To the tissue paper (core wrap sheet 41) is added a paper strong agent in terms of achievement of good strength property (tensile strength). The paper strong agent includes a dry paper strong agent, which improves the strength of a dry paper, and a wet paper strong agent, which improve the strength of a wet paper. Any of them may be used. In particular, carboxymethyl cellulose (CMC), which is one of the dry paper strong agents, is preferably used in the present invention, because it is water-soluble, and it is easily handled.

As the dry paper strong agent, conventionally known dry paper strong agents can be used, and they may include, for example, carboxymethyl cellulose (CMC) and salts thereof, polyacrylic amide resins and salts thereof, cationized starch, polyvinyl alcohol (PVA), and the like. They may be used alone or as a mixture of two or more kinds thereof. As each of the salts of CMC and the polyacrylic amide resin, sodium salts are mainly used. The polyacrylic amide resins may include, for example, cationic or anionic polyacrylic amide (PAM). Of these dry paper strong agents, CMC and the salts thereof, and anionic PAM and the salts thereof are particularly preferable.

As the wet paper strong agent, conventionally known wet paper strong agent can be used, and they may include, for example, polyamide polyamine epichlorohydrin (PAE), a urea-formalin resin, a melamine-formalin resin, dialdehyde starch, polyethyleneamine, methlolated polyamide, and the like. They may be used alone or as a mixture of two or more kinds thereof. Of these wet paper strong agents, PAE is particularly preferable.

The addition amount of the paper strong agent added in the tissue paper is preferably from 0.01 to 1.5% by mass, based on the dry mass of the total configuration fibers in the tissue paper, more preferably from 0.03 to 1.2% by mass. When the addition amount of the paper strong agent is too small, satisfactory strength property including the tensile strength cannot be obtained; on the other hand, when the addition amount of the paper strong agent is too large, the formation of the tissue paper may be deteriorated due to sticking of the paper to a Yankee dryer or adhesion of the paper strong agent to a mesh drum at the time of the production of the tissue paper, in addition to the hardening of the tissue paper (reduced texture).

The tissue paper may include other components in addition to the fibers such as NBKP and the paper strong agent described above. The other components may include, for example, additives generally used for raw materials for paper-making, such as a loading materials including talc, a dye, a color pigment, an antibacterial, a pH-controlling agent, a retention aid, a water resistant additive, an antifoaming agent, and the like. They may be used alone or as a mixture of two or more kinds thereof.

The tissue paper can be produced in a known wet paper-making process. The wet paper-making process includes a paper stock preparing step in which paper stock (slurry) including an aqueous dispersion of fibers such as NBKP is prepared, and a paper-making step in which fibrous webs are taken out from the paper stock and the webs are dried while it is carried. The paper-making step is usually divided into a wire part, a press part, a dryer part, a size press, and a calendaring part, the implementation is carried out sequentially. The paper strong agent described above is usually added to the paper stock in the paper stock preparing step. The wet paper-making process can be carried out in accordance with a usual manner using, for example, a paper-making machine such as a fourdrinier paper machine, a twin wire paper machine, an on-top paper machine, a hybrid paper machine or a cylinder paper machine.

The basis weight and the density of the tissue paper (core wrap sheet 41) are set at comparatively low values, in terms of the improvement of the liquid-permeability. Specifically, the basis weight is from 10 to 14.5 g/m², preferably from 11 to 14 g/m²; and the density is from 0.05 to 0.2 g/cm³, preferably from 0.1 to 0.2 g/cm³. If the basis weight and the density is set at the low values as above, the reduction of the paper strength is concerned. According to the present invention, however, as described above, such concern is overcome by using the NBKP having the freeness within the specific range, and using the paper strong agent at the same time. When the tissue paper has a basis weight of less than 10 g/m² or a density of less than 0.05 g/cm³, the paper strength may be remarkably reduced. On the other hand, when the tissue paper has a basis weight of more than 14.5 g/m² or a density of more than 0.2 g/cm³, the effect of improving the liquid-permeability may be poor.

The basis weight of the tissue paper is measured as follows: Under conditions described in JIS P 8111, after a sample (tissue paper) is subjected to humidification, a 10 square cm (area: 100 cm²) specimen to be measured is cut from the sample, and a weight of the specimen to be measured is measured with a balance measuring up to two decimal places. The measured value is divided by the area to calculate the basis weight of the specimen to be measured. Basis weights are calculated for ten specimens to be measured, which are cut from the sample, and an average thereof is defined as the basis weight of the sample.

The density of the tissue paper is measured as follows: After ten 20 square cm samples (tissue paper) are placed one upon another to obtain a laminate, the laminate is solidified with cooling in liquid nitrogen, and a middle part of the laminate is cut with a cutter. Samples, which do not have shear on a cross-section generated by cutting with a cutter, are selected from the then samples, and a thickness of the sample selected is measured with the light microscope. When the sample has unevenness, such as a crepe described below, the thickness of the sample is not a length (apparent thickness) between the lowermost part and the uppermost part in the uneven part, but a length (real thickness) of a part in which the configuration fibers are accumulated. A weight of the 20 square cm sample, whose thickness is measured as above, is measured using a balance measuring up to two decimal places. The target density is calculated by dividing the weight of the sample by a volume obtained by calculating from the thickness of the sample.

The tissue paper (core wrap sheet 41) may have crepes (fine wrinkles). The crepe on the tissue paper may be a wet crepe, which is generated when a wet fibrous web (tissue paper) is detached from a press roll with a doctor knife, or the like, in a press part in the paper-making step during the production of the tissue paper, or may be a dry crepe, which is generated when a dry fibrous web (tissue paper) is detached from a Yankee dryer with a doctor knife, or the like, in a dryer part. A crepe ratio can be obtained by the following equation: Crepe Ratio (%) = (A/B) × 100 - 100
wherein A shows a paper-making speed in the paper-making step, and B shows a take-up rotational speed of the paper.

The tissue paper having the crepes has a liquid-permeability higher than that of a tissue paper having no crepes, and the higher the crepe ratio, the higher the liquid-permeability. When the crepe ratio becomes high, however, the strength property (tensile strength) tends to be reduced. The crepe ratio of the tissue paper (core wrap sheet 41) is preferably from 5 to 30%, more preferably from 5 to 20%, particularly preferably from 7 to 15%, in terms of the balance between the liquid-permeability and the strength property.

Forming materials for each part of the diaper 1 will be explained. Various materials, which have been conventionally used in the concerned technical field, may be used as the backsheet 3. A sheet which is liquid-impermeable and moisture-permeable can be used as the backsheet 3, such as resin films which have moisture-permeability obtained by providing micropores piercing the sheet in the thickness direction; non-woven fabrics such as water-repellent non-woven fabrics; and laminates thereof with another sheet. The same sheet as the backsheet 3 can be used as the side sheet 62.

As the absorbent core 40, for example, fiber assemblies formed of hydrophilic fibers such as wood pulps or synthetic fibers, or the fiber assemblies having absorbent polymer particles can be used. The absorbent core 40 has a density of, preferably 0.03 to 0.2 g/cm³, more preferably 0.06 to 0.15 g/cm³. When the density of the absorbent core 40 is within the range described above, a soft absorbent member 4 having excellent absorption property can be obtained more reliably.

The diaper 1 in the first embodiment is used in the same manner as in a case using a known open type disposable diaper. In the diaper 1 of the first embodiment, the characteristic structure is adopted in each of the topsheet 2, the absorbent core 40 and the core wrap sheet 41, described above, in other words, 1) the topsheet 2 having the uneven shape on the skin-facing surface, 2) the absorbent core 40 having the slit 5 extending in the longitudinal direction X, and 3) the core wrap sheet 41 having a liquid permeation time, as measured by the measurement method described above, of 2 seconds or shorter, are adopted and, therefore the absorbency to excrement liquid such as urine or loose stool is excellent and it is difficult that the excrement liquid adheres to the skin of a wearer, thus resulting in reduced occurrence of rough skin. In particular, in the topsheet 2, while the uneven shape inhibits the diffusion of the comparatively high viscosity excrement liquid such as loose stool in the surface direction, the excrement liquid is pulled quickly into core wrap sheet 41 side, whereby the number of chance to contact the excrement liquid with the skin of the wearer immediately after the excretion is reduced; in the core wrap sheet 41, the excrement liquid such as urine or loose stool is quickly permeated into the absorbent core 40 side, and the excrement liquid is promptly moved to a position far away from the skin of the wearer; and in the absorbent core 40, the action of the slit 5 enhances the absorbency to the excrement liquid such as loose stool as a whole, and thus the occurrence of wet back can be effectively prevented. In short, in the diaper 1 of the first embodiment, the number of the contact chances of the comparatively low viscosity excrement liquid such as urine with the skin and the amount of the excrement liquid adhering to the skin are reduced by the improved absorbency to the excrement liquid immediately after the excretion and the reduced number of the occurrence of the wet back, the swelling of the skin, which is caused by the adhesion of the excrement liquid to the skin, is prevented and, at the same time, the number of the contact chances of excrement liquid such as loose stool, which may stimulate the swollen skin, with the skin and the adhesion amount thereof are also reduced, whereby the rough skin can be effectively prevented.

One of the effects obtained from the slit 105 in the diaper 101 of the second embodiment includes the improvement of the fit to a body of a wearer of the diaper 101. In the diaper 101 (absorbent core 140), in particular, an area from the front portion B to the crotch portion C (front area of the diaper 101) where the slits 105 exists in a deviated state, as shown in FIG. 9, when the diaper is compressed in the width direction Y by femurs of a wearer 110 in a state in which the wearer wears the diaper 101, a portion located outwardly in the width direction Y from the 2 slits 105 and 105 extending in the longitudinal direction X (outer part) is bent to the non-skin-facing surface side of the diaper 101 at the slit 105 as a line of bending (so that the line of bending lies on the slit 105), whereby the diaper 101 deforms so that it follows and tightly adheres to the skin 110a of the crotch portion of the wearer 110, thus resulting in achievement of good fit. The slit 105, therefore, functions as a flexible axis of the diaper 101 (absorbent core 140).

Even if the slit 105 is a pierced slit, which pierces the absorbent core 140 in the thickness direction, as the opening described in JP 2007-215688 A, it is considered that the slit can exert the function as the flexible axis described above. In such a case, however, the continuity of the absorbent core is interrupted at the pierced slit, and thus disadvantages such as reduced shape retention of the absorbent core and inhibition of the liquid diffusibility or rewettability may occur. On the other hand, the slit 105 according to the second embodiment is, as described above, the non-pierced slit, and in a portion adjacent to the slit 105 in the thickness direction of the absorbent core 140 on the absorbent core 140, the materials forming the absorbent core 140 are accumulated to form the portion facing the opening 140c, and thus the continuity of the absorbent core 140 is maintained, and thus the disadvantage described above does not occur.

In addition, even if there is the portion facing the opening 140c, in a case in which the portion is consolidated [a case in which the portion facing the opening 140c has a density higher than that of other portions except for the silt 105 in the absorbent core 140 (for example, a peripheral part of the portion facing the opening 140c described below)], the portion facing the opening 140c, which is consolidated, is high in the rigidity and hard, and thus it is difficult to bend the diaper 101 (absorbent core 140) at the hard portion facing the opening 140C and the slit 105 adjacent thereto in the thickness direction; as a result, a line of bending is formed at a position which deviates from the slit 105 and the diaper is bent at that position, i.e., there is a case in which the diaper is not bent as planned. In such a case, the effect of improving the fit may not be obtained as expected. The portion facing the opening 140c in the second embodiment, however, is not consolidated as described above, and the density D1 of the portion facing the opening 140c is the same as or lower than the density D2 of the peripheral part of the portion facing the opening 140c on the absorbent core 140 (an area within 5 mm from the portion facing the opening 140c on the absorbent core 140 except for the slit 105), thus resulting in no risk in which the bending at the portion facing the opening 140c is inhibited.

The reason why the portion facing the opening 140c, which is not consolidated, has a density lower than that of the peripheral part is as follows: In a carrying step after the slit 105 (portion facing the opening 140c) is formed during the production of the absorbent core 140, when a pressure is applied to the whole absorbent core 140 in the thickness direction by various rolls such as a carrying roll, the peripheral part of the portion facing the opening 140c may be compressed to some extent by that pressure, thus resulting in the increased density; however, the portion facing the opening 140c is smaller in a real thickness (a thickness of the absorbent core 140-forming materials accumulated) and is thinner, compared to the peripheral part, and thus the pressure is applied to the portion rarely, and the portion is not substantially compressed. During the production of the absorbent core 140, therefore, the density of the portion facing the opening 140c is not substantially changed, but the density of the peripheral part thereof may be increased by the compression while it is carried, and in such a case, the density of the portion facing the opening 140c becomes relatively lower.

Other one of the effects obtained from the slit 105 in the diaper 101 of the second embodiment includes the improvement of the absorbency to the excrement liquid. When the slit 105, which has substantially no materials forming the absorbent core 140 and extends in the longitudinal direction X of the absorbent core 140, is formed on the absorbent core 140, the excrement liquid such as loose stool, which is locally excreted into the portion facing the excretory part of the crotch portion C, promptly diffuses in the longitudinal direction X of the absorbent core 140 through the slit 105 as a conduction path, and thus an area at which the excrement liquid is substantially absorbed on the absorbent core 140 is increased, whereby the wet back of urine or loose stool (a phenomenon in which excrement liquid permeating the topsheet 102 is not kept in the absorbent member 104 and returns to the topsheet 102) can be reduced. In particular, in the excretion at an early stage, the slit 105 functions as a reserve part for temporarily storing the excrement liquid at the early state, and thus the absorbency to the excrement liquid such as loose stool is improved.

In addition, in the second embodiment, the portion facing the opening 140c, which is formed by accumulating the materials forming the absorbent core 140 and is not consolidated, is formed on the skin-facing surface 140a side of the portion at which the silt 105 is formed on the absorbent core 140, and thus the excrement liquid, which reaches the skin-facing surface 140a of the absorbent core 140 after permeating through the topsheet 102 and the core wrap sheet 141 in order, is diffused at the portion facing the opening 140c in the width direction Y, whereby it is possible to utilize the absorbent core 140 as a whole for absorbing and keeping the excrement liquid, and the effect of reducing the wet back of urine or loose stool can be more improved.

If there is no portion facing the opening 140c, in other words, if the slit 105 pierces the absorbent core 140 in the thickness direction, the pierced slit 105 divides the diffusion of the excrement liquid in the width direction Y, and thus the improvement effect of utilization efficiency of the absorbent core 140 cannot be really expected. Even if there is the portion facing the opening 140c, when it is consolidated [a case in which the portion facing the opening 140c has a density higher than that of other portions of the absorbent core 140 except for the slit 105 (for example, the peripheral part of the portion facing the opening 140c)], the liquid is not quickly diffused, thus resulting in the delayed diffusion of the liquid in the consolidated part, and thus the diffusion effect of the excrement liquid in the width direction Y cannot be similarly obtained.

In terms of the reliable exertion of the effects obtained by the slit 105, as described above, slit 105 has a width W104 (see FIG. 7) of preferably 1 to 20 mm, more preferably 1 to 10 mm. The width W4s, which is the narrowest in the width W4 of the slit 105, is preferably from 1 to 10 mm, more preferably from 1 to 5 mm. From the same viewpoint, a ratio of a total W50 of the narrowest widths W4s of all slits 105 on the crotch portion C (the portion facing the excretory part described above) to a total width W0 in the width direction Y of the absorbent core 140 on the crotch portion C (W50/W0) is preferably from 0.02 to 0.3, more preferably from 0.05 to 0.25.

Forming materials for each part of the diaper 101 will be explained. Various materials, which have been conventionally used in the concerned technical field, may be used as the topsheet 102 and the backsheet 103. As the topsheet 102, various liquid-permeable sheet materials such as non-woven fabrics and films having opening can be used. Various liquid-impermeable or water-repellent sheet can be used as the backsheet 103, such as non-moisture-permeable resin films, moisture-permeable resin films having micropores, non-woven fabrics such as water-repellent non-woven fabrics, and laminates thereof with another sheet. The same sheet as the backsheet 103 can be used as the side sheet 162.

As the absorbent core 140, for example, fiber assemblies formed of hydrophilic fibers such as wood pulps or synthetic fibers, or the fiber assemblies having absorbent polymer particles can be used. The absorbent core 140 has a density of, preferably 0.03 to 0.2 g/cm³, more preferably 0.06 to 0.15 g/cm³. When the density of the absorbent core 140 is within the range described above, a soft absorbent member 104 (absorbent core 140) having excellent liquid absorbency can be obtained. As the core wrap sheet 141, for example, a paper such as tissue paper, various non-woven fabrics, and liquid-permeable sheet materials such as films having opening can be used.

The diaper 101 in the second embodiment is used in the same manner as in a case using a known open type disposable diaper. The diaper 101 of the present embodiment causes flexibly bending deformation at the slit 105 as the line of bending, as shown in FIG. 9, due to the function of the slit 105 as a flexible axis, when it is worn, and thus the fit to the body of the wearer is excellent. In addition, the diaper 101 of the second embodiment is excellent in the absorbency to the excrement liquid due to the liquid-permeation and diffusion function of the slit 105 to the excrement liquid, as described above, and thus the high leakage prevention property can be exhibited together with the improved leakage prevention property caused by the improvement of the fit.

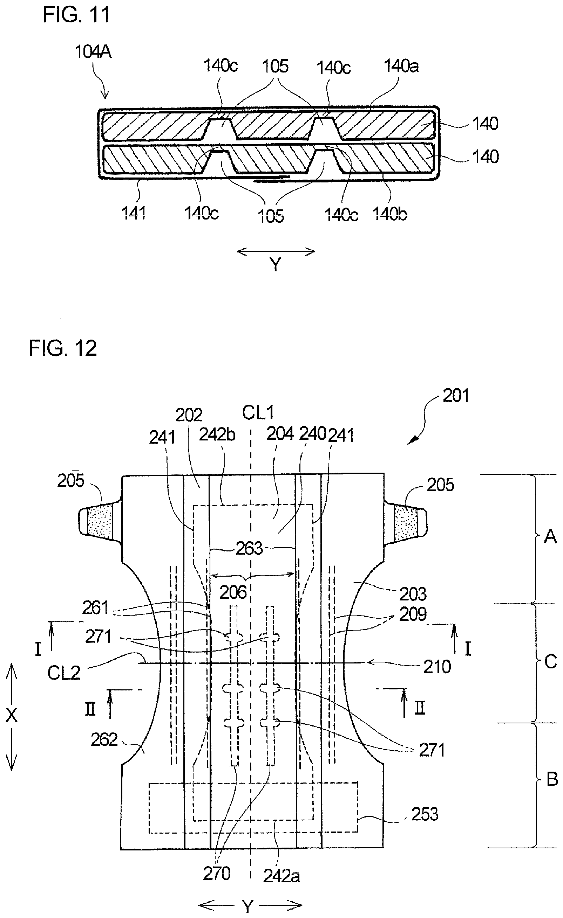

FIG. 10 and FIG. 11 show principal parts of absorbent articles of the present invention in other embodiments. For the other embodiments described below (embodiments concerning FIG. 10 and FIG. 11), configuration parts different from those in the second embodiment described above are mainly explained, and the same configuration parts are shown by the same reference signs and the explanations thereof are not repeated. The same explanations as in the second embodiment described above are suitably applied to configuration parts which are not particularly explained.

FIG. 10 (a) and FIG. 10 (b) show slits of other embodiments according to the present invention. A slit 105A shown in FIG. 10 (a) has a portion in which the width of the slit 105A is gradually increased from the skin-facing surface 140a side to the non-skin-facing surface 140b side of the absorbent core, similar to the slit 105 in the embodiment described above, whose shape is a trapezoid in a section view, and there is the opening 150 of the slit 105A on a side at which the width of the slit 105A is relatively wide. The slit 105A has a shape in which a projection in a (isosceles) trapezoidal shape is connected to a semicircular apex part in a section view in the width direction Y.

On the other hand, a slit 105B shown in FIG. 10 (b) has a (isosceles) trapezoidal shape in the cross-section of the width direction Y, as the slit 105 in the embodiment described above, the change in the width is opposite to that of the slit 105. Thus, the slit 105B has a portion in which the width thereof is gradually decreased from the skin-facing surface 140a side to the non-skin-facing surface 140b side of the absorbent core, and there is the opening 150 of the slit 105B on a side at which the width of the slit 105B is relatively wide. There is a portion facing the opening 140c shown in FIG. 10 (b) on the non-skin-facing surface 140b side of the absorbent core 140, the portion is closer to a backsheet 103 (not shown in FIG. 10) than an opening 150.

FIG. 11 shows an absorbent member of another embodiment according to the present invention. The absorbent member 104 in the embodiment described above has a monolayer structure including one layer of the absorbent core 140, but an absorbent member 104A shown in FIG. 11 has a multilayered structure including a plurality of layers (two layers) of the absorbent core 140. The absorbent article formed by including the absorbent member 104A can exert the same effects as in the embodiment described above.

The present invention is not limited to each embodiment described above, and suitable modification can be made without departing from the scope of the present invention. For example, in the first embodiment described above, the topsheet 2 has the multilayered structure including the upper layer 20 and the lower layer 21, and the laminate of a plurality of sheets, but it may has a monolayer structure including one sheet. In addition, in the embodiment described above, the elastic member 64 is arranged on the waist portion (end in the longitudinal direction X) at each of the rear portion A and the front portion B to form the waist gather, but there may be no waist gather (elastic member 64).

In addition, in the first embodiment, the slit 5 extending in the longitudinal direction X of the absorbent core 40 deviates to the rear portion A side including the crotch portion C, but it may be formed only on the crotch portion C, or it may deviates to the front portion B side including the crotch portion C, or it may be continuously formed throughout the length in the longitudinal direction X, but does not deviate to the specific portion in the longitudinal direction X of the absorbent core 40.

In the second embodiment, the slit 105 extending in the longitudinal direction X of the absorbent core 140 deviates to the front portion B side including the crotch portion C, but it may be formed only on the crotch portion C, or it may deviates to the rear portion A side including the crotch portion C, or it may be continuously formed throughout the length in the longitudinal direction X, but does not deviate to the specific portion in the longitudinal direction X of the absorbent core 140.

The number of the slits 5 or 105 is not particularly limited, and for example, one slit may be formed at the center in the width direction Y of the absorbent core 140 (on a virtual straight line dividing the absorbent core 140 in two in the width direction Y). The number of the slits 5 or 105 is not particularly limited, and for example, one slit may be formed at the center in the width direction Y of the absorbent core 40 or 140 (on a virtual straight line dividing the absorbent core 40 or 140 in two in the width direction Y). The cross-sectional shape of the slit 5 or 105 in the width direction Y is not limited to the trapezoidal shape, as shown in FIG. 2 or FIG. 8, may be a triangular shape, a square shape, a rectangular shape, or the like.

It is enough that the slit in the present invention has the opening at least on the non-skin-facing surface side of the absorbent core, and it may pierce the absorbent core in the thickness direction, and it may have the opening on the skin-facing surface side of the absorbent core in addition to the non-skin-facing surface side. The extending direction of the slit in the present invention is not limited to the longitudinal direction of the absorbent core (absorbent article), and may be the width direction.

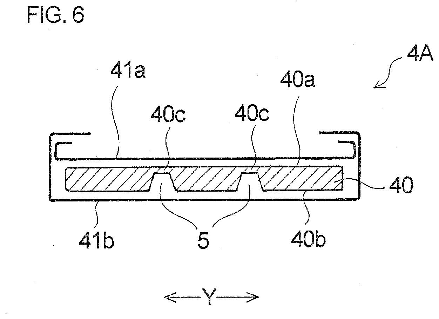

In the first and second embodiments, the core wrap sheet 41 or 141 is one continuous sheet, and the sheet is bent along the absorbent core 40 or 140 to cover the whole area of the surface of the absorbent core 40 or 140 except for the both end surfaces in the longitudinal direction X of the absorbent core 40 or 140, but instead of this, using two core wrap sheets, the skin-facing surface of the absorbent core may be covered with the one sheet and the non-skin-facing surface may be covered with the other sheet. For example, as shown in FIG. 6, two core wrap sheets 41a and 41b may be used. In the absorbent member 4A shown in FIG. 6, a core wrap sheet 41a covers the whole area of the skin-facing surface 40a of the absorbent core 40, and a core wrap sheet 41b covers the whole area of the non-skin-facing surface 40b of the absorbent core 40. The core wrap sheet 41b extends outwardly in the width direction Y from the both side edge parts along the longitudinal direction X of the absorbent core 40, and the extending part is rolled above the core wrap sheet 41a, which is located above the skin-facing surface 40a of the absorbent core 40. The absorbent core 40 and the core wrap sheet 41a or 41b may be joined with a known joint means such as a hot-melt adhesive, respectively.

A sub-layer sheet may be arranged between the topsheet 2 or 102 and the absorbent member 4 or 104. The sub-layer sheet enhances the liquid absorption property of the diaper 1 or 101, and provides the effect of reducing the phenomenon in which the liquid absorbed in the absorbent member 4 or 104 returns to the topsheet 2 or 102 side (wet back). As the sub-layer sheet, a sheet mainly including hydrophilic fibers such as a wood pulp (a sheet having a content of the hydrophilic fibers of preferably 90% by mass or more) can be used. The sheet may include, for example, a paper, a non-woven fabric, webs, and the like. The arrangement form of the sub-layer sheet is not particularly limited, and it may be arranged on an area from the rear portion A to the crotch portion C, or may be arranged on an area from the front portion B to the crotch portion C, or may be arranged only on the crotch portion C.

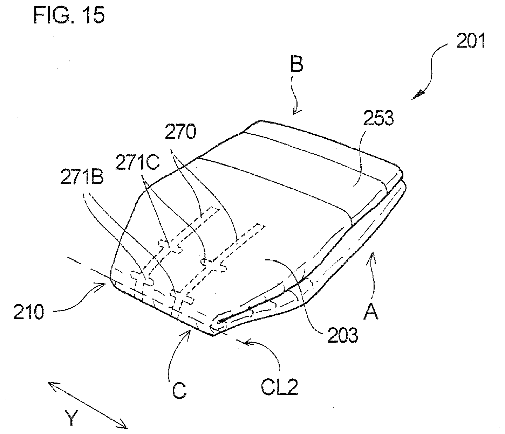

A disposable diaper 201, which is a third embodiment of the present invention (hereinafter referred to as sometimes the diaper 201) is folded in two in the longitudinal direction at a position of a center line CL2 in the longitudinal direction, as shown in FIG. 15. The diaper 201 includes, as shown in FIG. 12, a liquid-permeable topsheet 202, a liquid-impermeable or low permeate backsheet 203, and a liquid-retentive absorbent core 240 located between the both sheets, and has a rear portion A, a front portion B, and a crotch portion C located between them in the longitudinal direction. In the absorbent core 240, a plurality of non-pierced slits 270, which each extend in the longitudinal direction of the diaper 201, are formed. The slits 270 and 270 are not consecutively connected to each other at least on the crotch portion C, and has a wide portion 271 having a wide slit width at a position different from the folded position 210 at which the article is folded in two.

In the third embodiment, the longitudinal direction of the diaper 201 refers to a direction corresponding to a front-back direction of a wearer when the diaper is worn, which is the X direction in FIG. 12. The width direction of the diaper 201 refers to a direction perpendicular to the longitudinal direction of the diaper 201 when the diaper is unfolded into a planar shape, as shown in FIG. 12, which is the Y direction in FIG. 12.

The center line CL1 in the width direction refers to a line which extends in the X direction and divides the diaper 201 into two equal parts in the width direction (divides a length of the diaper 201 in the Y direction into two equal parts); and the center line CL2 in the longitudinal direction refers to a line which extends in the Y direction and divides the diaper 201 into two equal parts in the longitudinal direction.

The folded position of the article folded in two is a folded position of the diaper folded in two, and, in the third embodiment, a folded position 210 is located at the same position as the center line CL2 in the longitudinal direction. When the diaper is folded in three, the folded positions of the article are at two positions which are distantly-positioned on the diaper in the longitudinal direction. In usual, the one is located on the front portion side from the center line CL2 in the longitudinal direction, and the other is located on the rear portion side from the center line CL2 in the longitudinal direction.

The front portion B is a portion which is arranged on a front side of a wearer when it is worn, the rear portion A is a portion which is arranged on a rear side of the wearer, and the crotch portion C is arranged between the front portion B and the rear portion A in the longitudinal direction (X direction).

In the third embodiment, the front portion B, the crotch portion C and the rear portion A are areas when the diaper 201 is divided into three in the longitudinal direction (X direction).

The diaper 201 of the third embodiment is, as shown in FIG. 12, an open type disposable diaper. Fastening tapes 205 and 205 are provided on the both side edge parts of the rear portion A, and a landing zone 253, where the fastening tapes 205 and 205 are fixed, is provided on an outer surface of the front portion B.

The diaper 201 is formed in the shape of an arc at the both side edges of the crotch portion C, and the central part in the longitudinal direction is constricted as a whole to form a sandglass-shape.

The backsheet 203 has a sandglass-shaped external form, which corresponds to the external form of the diaper. The topsheet 202 has a width narrower than that of the backsheet 203, and is arranged on a central part in the width direction of the backsheet 203. A long absorbent member 204 is arranged between the topsheet 202 and the backsheet 203 in the same direction (X direction) as the longitudinal direction of the diaper 201. The absorbent member 204 is formed in the shape of an almost arc at the both side edges of the crotch portion C, and has a sandglass-shape in a planar view as a whole, whose central part in the longitudinal direction is constricted. The topsheet 202 and the backsheet 203 each extend outwardly from the both side edges and the both end edges of the absorbent member 204, and their extending parts are joined to each other.

The absorbent member 204 includes an absorbent core 240, which is formed from a fiber assembly made of fiber materials such as defibrated pulp or the fiber assembly to which a functional materials such as absorbent polymer particles are added, and a core wrap sheet, which covers the absorbent core 240. As the core wrap sheet, various known ones may be used, and, for example, thin papers such as tissue paper and water-permeable non-woven fabrics are preferably used. The absorbent core 240 has the almost same shape as that of the absorbent member 204 in the planar view. The both side edges of the absorbent member 204 are located at the almost same positions as those of the both side edges 241 and 241 of the absorbent core 240. The core wrap sheet has end edges which are located at the same positions as those of the end edges 242a and 242b in the longitudinal direction of the absorbent core 240, or at positions at which it extends in the X direction from the both end edges 242a and 242b.

On the both sides on the crotch portion C of the diaper 201, leg portion elastic members 209 are arranged in a stretched state. The plurality of leg portion elastic members 209 are arranged on each side of the diaper 201 in the longitudinal direction thereof. The leg portion elastic members 209 are arranged between the backsheet 203 and a sheet material 262 for forming three-dimensional gathers 206 and 206 in a fixed state with an adhesive, along the side edges 241 and 241 of the absorbent member 204 in the Y direction outwardly from the positions of the side edges 241 and 241 of the absorbent member 204. The leg portion elastic members 209 are located at a leg portion which is arranged around a leg of a wearer, and forms a leg gather at the leg portion.

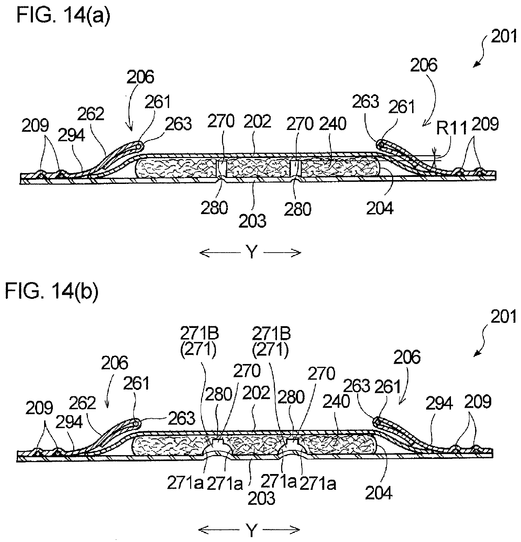

As shown in FIG. 12 and FIG. 14, a pair of three-dimensional gathers 206 and 206 are formed on both sides of the diaper 201 in the longitudinal direction. Each three-dimensional gather 206 is formed by arranging the sheet material 262 for forming the three-dimensional gather having the elastic member 261 on the both sides in the longitudinal direction of the diaper so that it extends from the outside to the inside of the both side edges of the topsheet 202 and fixing it. The three-dimensional gather 206 has a linear fixed end 294, which extends in the X direction, at a vicinity of the side edge of the absorbent core 240, and a free end 263 on the center line CL1 side in the width direction. The both sides in the longitudinal direction of the backsheet 203 extend outwardly in the width direction from the both side edges of the topsheet 202, and the sheet material 262 for forming the three-dimensional gather is laminated on and bonded to the extended part thereof. On the sheet material 262, a plurality of threadlike elastic members 261 are arranged in almost parallel to the free end 263, and the three-dimensional gather 206 stands toward a skin of a wearer by shrinking the elastic members 261, when the diaper is worn.

In the diaper 201 of the third embodiment, two slits 270, which extend in the longitudinal direction (X direction), are formed on the absorbent core 240. The two slits 270 and 270 have the same length in the X direction, and are parallel to the X direction. The two slits 270 and 270 are formed at an interval in the Y direction, and have symmetrical shapes to the center line CL1 in the width direction.

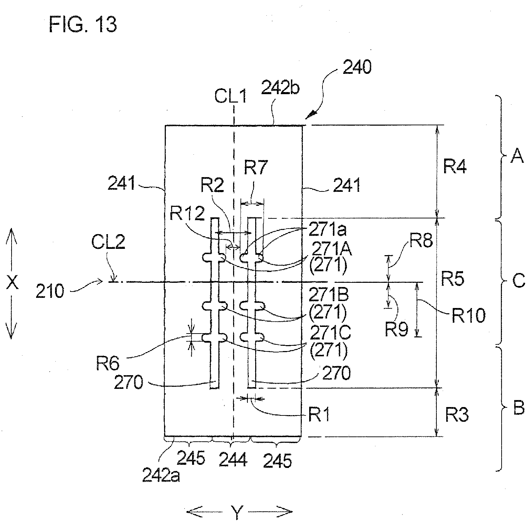

As shown in FIG. 14, the absorbent core 240 in the diaper 201 has an almost uniform thickness except for portions other than the slits 270. In addition, a slit width R1 (a size in the direction, see FIG. 13) of the slit 270 is almost constant along the X direction except for portions other than wide portions 271A to 271C described below.

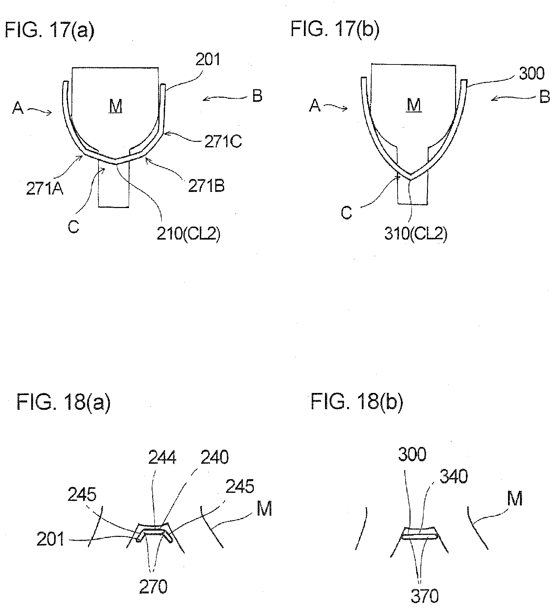

The slit width R1 of portions other than the wide portions 271A to 271C in the slit 270 is preferably from 1 to 10 mm, more preferably from 1 to 5 mm, in terms of realizing a good bending by the slit [see FIG. 18 (a)] in the width direction, which is obtained by the slit. The separation distance R2 (the distance between the centers, see FIG. 13) in the Y direction of the slits 270 and 270 is preferably from 10 to 70% of the width of the absorbent core 240 (distance between the side edges 241 and 241) on the center line CL2 in the longitudinal direction, more preferably from 20 to 50%. The separation distance R2 (the distance between the centers, see FIG. 13) is preferably from 10 to 50% of the total width of the absorbent core 240 (the maximum width in the Y direction, in an example shown in FIG. 12, a distance between the side edges 241 and 241 at the both ends in the longitudinal direction of the absorbent core 240), more preferably from 20 to 30%. In a case of diapers for babies, the separation distance R2 is preferably from 10 to 50 mm, particularly preferably from 20 to 30 mm.

The absorbent core 240 has a distance R3 (see FIG. 13) in the X direction from an end edge 242a of a front portion B side (hereinafter may be referred to as the front side) to a front side end of the slit 270 of preferably 10 to 40% of the full length of the absorbent core 240 in the X direction, particularly preferably 20 to 30%. When the distance R3 is set within the number range described above, the slit 270 easily extends until an area where the diaper adheres tightly to the wearer on the front side.

In addition, the absorbent core 240 has a distance R4 (see FIG. 13) in the X direction from an end edge 242b of a rear portion A side (hereinafter may be referred to as the rear side) to a rear side end of the slit 270 of preferably 15 to 45% of the full length of the absorbent core 240 in the X direction, particularly preferably 30 to 40%. When the distance R4 is set within the number range described above, the slit 270 easily extends until an area where the diaper adheres tightly to the wearer on the rear side.

The full length of the absorbent core 240 in the X direction is preferably, for diapers for babies, from 200 to 500 mm, particularly preferably from 300 to 450 mm. The length R5of the slit 270 is preferably from 30 to 60% of the full length of the absorbent core 240.

When the slit 270 extends until the area where the diaper adheres tightly to the wearer on the front side and/or the rear side, the effect of improving the fit, which is obtained by the slit 270, can be exerted to the area where the diaper adheres tightly to the wearer on the front side and/or the rear side, and the excrement is diffused in the X direction, without retaining it in the crotch portion C; as a result, the absorption property of the absorbent core 240 in the X direction can be sufficiently exhibited as a whole.

In an example shown in FIG. 12, the slit 270 is formed longer in a portion located on the front side from the center line CL2 in the longitudinal direction than in a portion located on the rear side from the center line CL2 in the longitudinal direction, in the X direction, and the slit 270 extends until an area where the diaper adheres tightly to the wearer only on the front side.

The slit 270 has wide portions 271 (271A to 271C), which have a wide slit width in a part in the longitudinal direction. The wide portion 271 is a portion in which the slit width (a width in the Y direction) of the slit 270 is widened at a predetermined point in the longitudinal direction of the slit 270.

In the diaper 201 of the third embodiment, as shown in FIG. 13, projection 271a, which protrude right and left, are provided at three points in the longitudinal direction of the slit 270, thereby forming the wide portions 271 (271A to 271C). More specifically, a pair of projections 271a and 271a are formed at each of the three points in the X direction of the slit 270, so that the projections having the same projection width protrude on the both sides in the Y direction, and a tip end of the projection is formed in the state of an arc.

The wide portions of the two slits 270 and 270 are formed at the same position in the X direction, and accordingly the two wide portions, which face across the center line CL1 in the width direction, or the four projections forming the wide portions are located in the same straight line which extends in the Y direction.

A length R6 in the X direction of the wide portion 271 (a width of a base end in X direction width of the projection 271a, see FIG. 13) is preferably from 2 to 15 mm, more preferably from 2 to 7 mm. The length R6 of the wide portion 271 of 2 mm or more is preferable, because the length in the X direction of the wide portion 271 is secured to realize good bending of the absorbent core 240 or the absorbent member 204 in the longitudinal direction, or good bending of the absorbent core 240 or the absorbent member 204 in the width direction at the crotch. The length of 15 mm or less, particularly 7 mm or less is also preferable, because the width in the X direction of the wide portion 271 is inhibited, whereby the properties of the absorbent core 240 are not deteriorated.

The two slits 270 and 270 in the diaper 201 are not consecutively connected to each other and independently exist at least in the crotch portion C. More specifically, the slits 270 and 270 are not consecutively connected to each other in the full length of the slit 270 in the X direction. Therefore, the wide portion of the one slit 270 and the wide portion of the other slit 270 are not connected to each other in the central part in the width direction of the absorbent core 240, and a central part 244 in the width direction of the absorbent core 240, located between the slits 270 and 270, runs continuously in the X direction along the full length of the slit 270.

A length R7 (see FIG. 13) in the Y direction of each wide portion 271A to 271C is preferably from 5 to 45% of a width of the absorbent core 240 in the center line CL2 in the longitudinal direction (a distance between the side edges 241 and 241), more preferably from 5 to 20%.

A ratio (R7/R1) of a length R7 in the Y direction of the wide portion 271A to 271C (see FIG. 13) to the slit width R1 of the portions other than the wide portion 271A to 271C is preferably from 1.2 to 5, particularly preferably from 1.5 to 2.5.

In addition, a length R7 in the Y direction of the wide portion 271A to 271C (see FIG. 13) is preferably from 2 to 45% of a width of the absorbent core 240 on the portion in the X direction at which each wide portion 271 is located (a distance between the side edges 241 and 241), more preferably from 2 to 30%.

In addition, a separation distance R12 between the wide portions 271 and 271 of the adjacent slits 270 and 270 (see FIG. 13) is preferably from 10 to 50 mm, particularly preferably from 15 to 30 mm, in terms of the maintenance of the bending property in the width direction of the absorbent core 240 or the absorbent member 204, which is obtained by the slits 270 and 270. The separation distance R12 (see FIG. 13) is preferably from 10 to 70% of a width of the absorbent core 240 on the portion in the X direction at which each wide portion 271 is located (a distance between the side edges 241 and 241), more preferably from 20 to 50%.

As stated above, the slit 270 has the wide portion 271 at a position different from the folded position 210 at which the diaper is folded in two (i.e., a position which is not overlapped on the folded line at which the diaper 201 is folded in two).

More specifically, the diaper 201 of the third embodiment has the two wide portions 271B and 271C, which are located on the front side portion from the folded position 210, as the wide portion 271, and one wide portion 271A, which is located on the rear side portion from the folded position 210. The wide portion 271A is located on the vicinity of the rear side of the folded position 210, and the wide portion 271B is located on the vicinity of the front side of the folded position 210. It is preferable that the wide portion 271A and the wide portion 271B are located on a side opposite to the folded position 210 in the X direction, because the bending at the folded position 210 can be easily relieved by the wide portions 271A and 271B (a bending angle can be easily widened). As shown in FIG. 12 and FIG. 13, both of the wide portion 271A and the wide portion 271B are located on the crotch portion C. The wide portion 271C, which is located on the front side from the wide portion 271B, is located on the vicinity of the boundary between the front portion B and the crotch portion C.

In order to successfully adjust the rear side of the wearer to the diaper 201, a distance R8 between the wide portion 271A located on the rear side from the folded position 210 and the folded position 210 (see FIG. 13) is preferably from 2 to 20% of the full length of the absorbent core 240 in the X direction, more preferably from 5 to 10%.

In order to successfully adjust the front side of the wearer to the diaper 201, a distance R9 between the wide portion 271B located on the front side from the folded position 210 and the folded position 210 (see FIG. 13) is preferably from 2 to 20% of the full length of the absorbent core 240 in the X direction, more preferably from 5 to 10%. In addition, a distance R10 between the wide portion 271C and the folded position 210 (see FIG. 13) is preferably from 10 to 35% of the full length of the absorbent core 240 in the X direction, more preferably from 15 to 25%.

As shown in FIG. 14, the slit 270 in the third embodiment is a non-pierced slit (depression channel) which opens only on the backsheet 203 side, and the absorbent core 240 has a thin wall portion 280, which is formed on the topsheet 202 side of the slit 270.

As described above, the "slit" in the present invention may include long and narrow depression channels as in the third embodiment. The non-pierced slit 270 in the third embodiment is formed by, for example, accumulating starting materials (fiber materials, high absorbent polymer particles, and the like) in a mold in which long and narrow projections are provided on the bottom surface, and accumulation parts on the projections are formed into the thin wall portion 280. When the projections partly have a wide portions, the slit 270 has the wide portion 271.

In terms of the sure acquisition of the bending by the slit 270, a thickness R11 of the thin wall portion 280 (see FIG. 14) is preferably from 5 to 50% of a thickness of the absorbent core 240. The thickness of the absorbent core 240 is preferably from 2 to 10 mm.

The cross-sectional shape of the wide portion 271 in the diaper 201of the third embodiment is bawl-shaped, formed by a projection curve which protrudes on the topsheet 202 side, as the cross-sectional shape of the wide portion 271B shown in FIG. 14 (b). By this shape, a depth of the wide portion 271 is formed so that the depth becomes gradually shallower from the base end to the tip end in the projections 271a and 271a. A depth of the wide portion 271 at the base end of the projection 271a is formed slightly shallower than a depth of the central of the slit 270 in the width direction (the same depth as that of portions other than the wide portions of the slit). Instead of this shape, as the wide portion 271, a wide portion may be provided in which the depth at the base end of the projection 271a is the same depth as that of the central part of the slit 270 in the width direction (the same as the slit depth of the portions other than the wide portions), or a wide portion may be provided in which the depth is uniform in the whole wide portion 271 area.

In the diaper 201 of the third embodiment, the backsheet 203 and the core wrap sheet described above (not shown) become depressed so that they get into the inside of the slit 270 along the inner surface of the slit 270.

The diaper 201 is folded at the folded position 210 in two in the longitudinal direction, whose backsheet 203 faces outside as shown in FIG. 15 in a packed state when it is put on the market, or the like.

The diaper 201 is put in a wrapping bag in the folded state and compressed. When it is used, the folded position 210 of the absorbent core 240 and the vicinity thereof are low in the rigidity in the longitudinal direction. In the diaper 201, it is preferable that the rigidity of the portion, in which the absorbent core 240 are arranged, in the longitudinal direction (X direction) of the diaper satisfies: G2 < G3 < G1 wherein G1 is a rigidity of a portion in which the slit 270 is not formed, G2 is a rigidity of a portion in which the wide portion 271 of the slit 270 is formed, andG3 is a rigidity of a portion in which portions other than the wide portion 271 of the slit 270 is formed. The same is applied to rigidities of the absorbent member 204 and the absorbent core 240 itself.

Specifically, when the rigidity is measured in a measurement method described below, it is preferable that the measured rigidities G1 to G3 are within the number range described below. G1 is preferably from 100 to 150 g. G3 is preferably from 80 to 130 g, and a difference thereof with G1 is preferably 10 g or more. G2 is preferably from 60 to 120 g, more preferably from 60 to 100 g. A difference between G2 and G3 is preferably from 10 to 70 g. A difference between G2 and G1 is preferably 20 g or more, more preferably 30 g or more. The difference between G2 and G1 of 30 g or more is preferable because the effects obtained by the wide portion 271 is surely obtained and thus the diaper 201 easily fits the wearer. The difference between G3 and G2 of 70 g or less is preferable, because the bending along the slit 270 is not disturbed by the wide portion.

### <Measurement Method of Rigidity>

### <Preparation of test piece>

A diaper, which has been folded, and put in a wrapping bag and compressed, was taken out from the wrapping bag, and gathers were removed from the diaper to make a sample for measurement of the width of the absorbent member.

### <Calculation Method of Rigidity>

Using a handle-o-meter, a softness of an absorbent member is evaluated. The softness evaluation using the handle-o-meter is performed by measuring the rigidity in accordance with the Japan Industrial Standards "JIS L-1096 (Testing Method for General Fabric)". The measured values obtained by the handle-o-meter show that the smaller the number, the easier the occurrence of the bending deformation described above, and the better the easy wearing and the fit. The measurement method using the handle-o-meter is as follows: A sample for measurement is put on a support having a channel with a width of 20 mm, the longitudinal direction of the sample being directed to a direction perpendicular to the channel. The longitudinal direction of the sample for measurement is the same direction as the longitudinal direction of the diaper. The sample for measurement is arranged so that a part where the rigidity is intended to be measured is located at a central part in the width direction of the channel (a position at which the distance between the one edge and the other edge is divided into a half). A 2-mm thick blade (a length in the same direction as that of the channel is more than the width of the absorbent member) is located at a center part in the width direction of the channel, the blade is fallen, and the sample for measurement is pushed into the channel. The maximum force, which is applied to the blade, is measured using a load cell when the blade is fallen a certain distance. An average value of values obtained at three points is defined as a measurement value. As the handle-o-meter, for example, a feeling tester (handle-o-meter method), HOM-3 Type, manufactured by Daiei Kagaku Seiki MFG. Co., Ltd, can be preferably used.

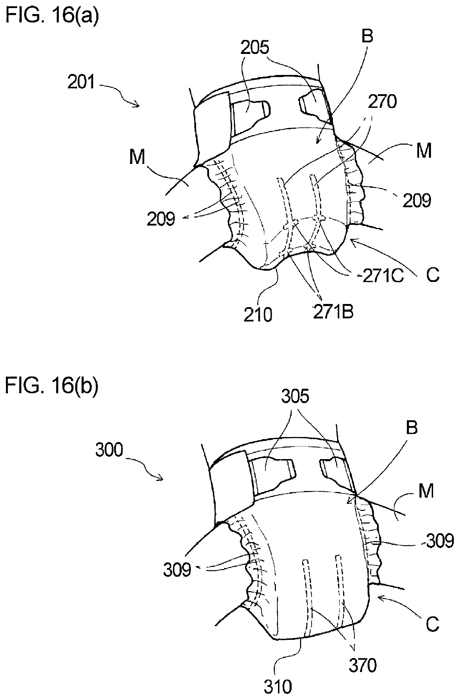

In the diaper 201 of the third embodiment, in the longitudinal direction of the absorbent member 204 (absorbent core 240), the portion having the wide portion 271 has a lower rigidity than that of the portion having no wide portion 271 of the slit 270, and thus the diaper is easily bent in the longitudinal direction. As shown in FIG. 16(a), when the diaper 201 is worn, the diaper 201 is easily bent in the longitudinal direction at each position of the wide portions 271A and 271A, the wide portions 271B and 271B, and the wide portions 271C and 271C. This results in that, as shown in FIG. 17 (a), the crotch portion C of the diaper 201 curves along the outer surface of a wearer M, the vicinity of the folded position 210 is lifted upwardly (on the wearer M side), and an angle of bend in the folded position 210 becomes greater. Keeping this state, as shown in FIG. 16 (a) and FIG. 18 (a), when a force is applied to the crotch portion C of the diaper 201 in the width direction by squeezing both legs of the wearer M, the absorbent core 240 is easily bent in the width direction along the slits 270 and 270, and the both sides 245 and 245 of the absorbent core 240 easily adhere tightly to a leg periphery of the wearer. In addition, as shown in FIG. 16 (a) and FIG. 18(a), in particular, when the absorbent core 240 is bent so that the central part 244 in the width direction of the absorbent core 240 protrudes on the wearer M side, the central part 244 thereof adheres to the wearer M. As a result, the diaper 201 of the embodiment is improved in the fit to the crotch of the wearer, and has excellent leakage prevention property.

On the other hand, as shown in FIG. 16 (b), a conventional diaper 300 is different from the diaper 201 of the third embodiment in that the wide portions are not provided in the slit 270. For this difference, as shown in FIG. 16 (b) and FIG. 17 (b), in the crotch portion C of the diaper 300, the angle of bend of the folded position 310 is not eased (increased) so much, when it is worn, and the crotch portion C of the diaper 300 fits insufficiently the outer surface of the wearer M. As shown in FIG. 16 (b) and FIG. 18 (b), if the wide portion C is squeezed by the both legs in this state, it is difficult to fold the absorbent core 240 along the slits 370 and 370 due to the obstruction caused by the fold of the folded position 310, and the fitting effect to the crotch by the slits 370 and 370 is insufficiently obtained.

As described above, according to the disposable diaper 201 of the third embodiment, even if a fold is generated, the effect of improving the fit to the crotch, which is obtained by the slits 270 and 270 in the longitudinal direction, is advantageously exhibited, and the excellent fit and leakage prevention property can be obtained.

In particular, in the diaper 201, because the slits 270 and 270 are not consecutively connected to each other in the crotch portion C, the bending along the slits 270 and 270 is not inhibited by a connected part.

The slits 270 and 270 are non-pierced slits which opens only on the backsheet 203 side, and when the diaper 201 is folded along the slit 270, it is easily folded, with the backsheet 203 side inside. Thus, as shown in FIG. 16 (a) and FIG 18 (a), when the diaper 201 is worn, it easily rises so that the central part 244 of the absorbent core 240 approaches the wearer M side, whereby the fit to the crotch can be more improved.

In the diaper 201, the wide portion 271B and the wide portion 271C are provided on the front portion B side from the folded position 210. In general, when a front portion side of a taping diaper is put on a wearer in the state in which the wearer lies down, it is difficult to apply the diaper to the wearer, because it is difficult to spread the front portion side due to shrinkage of a three-dimensional gather elastic member and a leg portion elastic member. According to the diaper 201 of the third embodiment, however, the front portion B side is easily spread because the low rigidity area, made of the wide portions, is provided on the front portion B side, and thus it is easy to wear the diaper along the abdomen of the wearer.

In addition, the diaper 201 has the plurality of wide portions 271B and 271C in the slits 270 and 270 on the front portion side from the position 210 at which the diaper is folded in two, and thus the front portion B side can be more easily spread when the taping diaper 201 is put on the wearer M in the state in which the wearer lies down, and it is easy to wear the diaper along the abdomen of the wearer M.

The more wide portions 271 are formed on the front portion B side than on the rear portion A side of the folded position 210, and thus the front portion B side can be more easily spread when the taping diaper 201 is put on the wearer M in the state in which the wearer lies down, and it is easy to wear the diaper along the abdomen of the wearer M.

With respect to materials for forming the diaper 201 in the third embodiment, various materials, which are conventionally used for absorbent articles such as disposable diapers and sanitary napkins, can be used, without particular limitations, for the topsheet 202, the backsheet 203, the elastic member 261 for forming the three-dimensional gather 206, the elastic member 209 for the leg portion, the absorbent core 240, and the like.

Next, a disposable diaper 201' will be explained. Points different from those in the third embodiment will be mainly explained, and the same points are shown by the same reference signs and explanations thereof are not repeated. The same explanations as in the disposable diaper 201 described above are suitably applied to points which are not particularly explained.

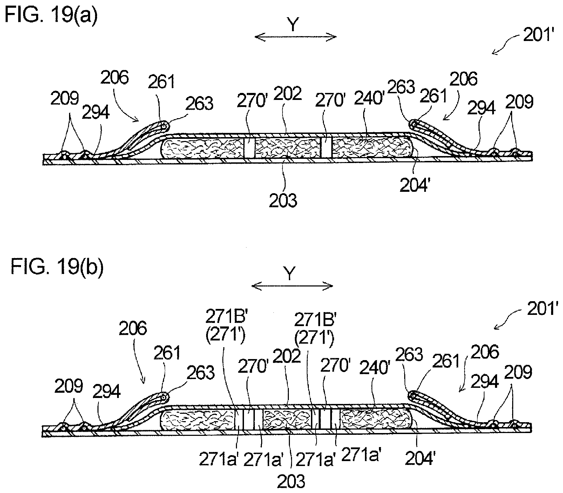

As shown in FIGS. 19 (a) and (b), in the diaper 201', a slit 270' opens both on a topsheet 202 side and on a backsheet 203 side, and accordingly it pierces an absorbent core 240' in the thickness direction. As shown in FIG. 19 (b), a wide portion 271' of the slit 270' (shown as a wide portion 271B' in FIG 19) pierces the absorbent core 240' in the thickness direction throughout the planar direction. Although only a cross-sectional shape of the wide portion 271B', which is formed on the same position as that of the wide portion 271B in the diaper 201, is shown in FIG. 19 (b), the same is applied to other wide portions 271A' and 271C', which are formed on the same positions of the wide portions 271A and 271C in the diaper 201. Thus, the slit 270' has the almost same shape of an opening which opens on the backsheet 203 side as the shape of an opening which opens on the topsheet 202 side, including the wide portion 271B'. A core wrap sheet, which covers the absorbent core 240', the topsheet 202 and the backsheet 203 have an almost planar shape at opening parts of the slit 270', and are not depressed so that they get into the inside of the slit 270'.

Although the diaper 201' is slightly inferior to the diaper 201 of the third embodiment in that the central part 244 in the width direction of the absorbent core 240 is easily bent so that it rises toward the wearer side, the almost same effects can be obtained as in the diaper 201 of the third embodiment.

The diaper of the third embodiment and diaper 201' disclosed above can be modified, for example, as described below.

For example, the planar view of the wide portion of the slit is not limited to that shown in FIG. 13, so long as a plurality of slits are not consecutively connected to each other.

In the diaper 201 described above, the three wide portions 271A to 271C have the same shape, which is not necessary. For example, the wide portion, which is located on a position at which the diaper is intended to be bent largely, may have a long length in the Y direction, and the wide portion, which is located on a position at which the diaper is intended to be bent small, may have a short length in the Y direction.

There are two slits in either of the absorbent cores 240 and 240', but the absorbent core may have three or more pierced or non-pierced slits, which extend in the X direction. For example, in a case of the three slits, two slits other than one central slit may divide the absorbent core into a central part in the width direction and both sides to form the central slit on a central part in the width direction. When the three slit are non-pierced slits, for example, two slits on the both sides may open on the backsheet side, and a central slit may open on the topsheet side, whereby the absorbent core 240 or the absorbent member 204 on the crotch portion C may easily be bent into an M-shaped cross-sectional view in the width direction, when the diaper is worn.

The wide portions are provided at three points in the slit of the diaper 201, but the number of the points, at which the wide portion is provided, may be one, two, four or more in the slit in the longitudinal direction. When the diaper is folded in two as in the embodiment, it is preferable to form one wide portion on each of the vicinity of the front side from the folded position and the vicinity of the rear side, in terms of good easing of the bending at the folded position.

The diaper 201 described above is folded in two, but it may be folded in three. In this case, the wide portion may be formed only at one point between the folded position on the front portion side and the folded position on the rear portion side, or may be provided on the outside in the longitudinal direction of the two folded positions. The portions may be provided at two positions or more. For example, they may be provided at front and back vicinities of each of the two folded positions.

The present invention can be applied to pull-on disposable diapers, which have been previously formed into pants, in addition to the open type disposable diapers having fastening tapes as in the embodiments described above, and further to sanitary napkins, pads for incontinence, and the like.

The absorbent article of the present invention may be pull-on disposable diapers for babies or adults or shorts sanitary napkins, in addition to the open type (taping) disposable diapers for babies or adults.

The features whose explanations are not repeated in one embodiment, and the features which exist in only one embodiment can each be applied to the other embodiments, and the features in each embodiment can be suitably changed between the embodiments.

The present disclosure also discloses absorbent articles described below.
<1> An absorbent article comprising an absorbent member and a topsheet which is arranged on a skin-facing surface side of the absorbent member, the absorbent member including an absorbent core and a core wrap sheet which covers at least a skin-facing surface of the absorbent core, wherein
   the topsheet has a large number of projections which protrude toward the skin of a wearer,
   the absorbent core has a slit extending in a predetermined direction and having an opening on at least non-skin-facing surface side of the absorbent core, and
   a portion of the core wrap sheet which covers the skin-facing surface of the absorbent core has a liquid permeation time of a low viscosity liquid, as measured in the following method, of 2.5 seconds or less.

### <Measurement Method of Liquid Permeation Time of Low Viscosity Liquid>

Two cylinders having an inside diameter of 35 mm, whose upper and lower ends are open, are arranged up and down bringing axis of the both cylinders in line, a sheet to be measured (core wrap sheet) is put between the upper and bottom cylinders, and, while that condition is kept, 40 g ± 1 g of physiological saline is supplied into the upper cylinder. The 40 g ± 1 g of physiological saline is poured into the upper cylinder of the physiological saline at once without pausing from a container including the physiological saline, thereby supplying the saline into the upper cylinder. The physiological saline supplied permeates through the sheet to be measured or is absorbed into the sheet to be measured, whereby it disappears from the upper cylinder. A time until a liquid surface of the physiological saline reaches a position of a surface of the sheet to be measured from the supply starting time of the physiological saline is measured, and the time is defined as a liquid permeation time.
<2> The absorbent article according to <1>, wherein the portion of the core wrap sheet which covers the skin-facing surface of the absorbent core has a liquid permeation time of a low viscosity liquid, as measured in the method described above, of 0.5 to 2.5 seconds.
<3> The absorbent article according to <2>, wherein the liquid permeation time of a low viscosity liquid, as measured in the method described above, is preferably from 0.5 to 2 seconds, more preferably from 0.5 to 1.5 seconds.
<4> The absorbent article according to any one of <1> to <3>, wherein the portion of the core wrap sheet which covers the skin-facing surface of the absorbent core has a liquid permeation time of a high viscosity liquid (mixed liquid of glycerol and deionized water), as measured in a method described below, of 500 seconds or less.

### <Measurement Method of Liquid Permeation Time of High Viscosity Liquid>

The measurement is performed in accordance with <Measurement Method of Liquid Permeation Time of Low Viscosity Liquid> described above, except that 10 g ± 1 g of high viscosity liquid having a viscosity of 290 mPa·s is used instead of 40 g ± 1 g of physiological saline. Using a digital pump (a trademark "Masterflex" manufactured by Cole-Parmer Instrument Company), 10 g ± 1 g of high viscosity liquid is supplied to the upper cylinder 91 at a speed of 100 g/ml, and a time until a liquid surface of the high viscosity liquid reaches a position of a surface of the sheet to be measured S (a surface on the upper cylinder 91 side) from the supply starting time of the high viscosity liquid is measured, and the time is defined as a liquid permeation time of the high viscosity liquid. The high viscosity liquid is prepared as follows. Glycerol and deionized water are mixed in a mass ratio of the former : the latter = 94:6, to which 0.01 g, based on the 100 g of the mixed liquid of Red No. 2 is added, thereby preparing the desired high viscosity liquid. The high viscosity liquid is acclimated in a thermostatic chamber at 23°C and 50% RH over one day before use.
<5> The absorbent article according to <4>, wherein the portion of the core wrap sheet which covers the skin-facing surface of the absorbent core has the liquid permeation time of a high viscosity liquid, as measured in the method described above, of preferably from 50 to 300 seconds, more preferably from 50 to 200 seconds.
<6> The absorbent article according to any one of <1> to <5>, wherein the core wrap sheet which covers the skin-facing surface of the absorbent core is a tissue paper including mainly a bleached Kraft pulp of a coniferous tree having a freeness of 400 to 550 ml, to which a paper strong agent is added.
<7> The absorbent article according to any one of <1> to <6>, wherein the core wrap sheet which covers the skin-facing surface of the absorbent core is a tissue paper having a basis weight of 10 to 14.5 g/m², and a density of 0.05 to 0.2 g/cm³.
<8> The absorbent article according to <7>, wherein the core wrap sheet which covers the skin-facing surface of the absorbent core is a tissue paper having a basis weight of 11 to 14 g/m², and a density of 0.1 to 0.2 g/cm³.
<9> The absorbent article according to any one of <1> to <8>, wherein the slit has a portion in which a width of the slit increases gradually from the skin-facing surface side toward the non-skin-facing surface side in the absorbent core.
<10> The absorbent article according to any one of <1> to <9>, wherein the slit does not pierce the absorbent core in a thickness direction, and a forming material of the absorbent core is accumulated between the slit and the portion of the core wrap sheet which covers the skin-facing surface of the absorbent core.
<11> The absorbent article according to any one of <1> to <10>, wherein the slit is a continuous straight line slit, the plurality of lines being formed at predetermined intervals in a lateral direction of the absorbent article.
<12> The absorbent article according to <11>, wherein a distance between the slits is from 10 to 50 mm.
<13> The absorbent article according to <11> or <12>, wherein the plurality of slits are parallel to each other, and the lengths thereof in the longitudinal direction are equal to each other.
<14> The absorbent article according to any one of <1> to <13>, wherein a position at which the slit is formed corresponds to an area in which loose stool is excreted.
<15> The absorbent article according to any one of <1> to <14>, wherein a portion of the absorbent core which is adjacent to the slit in a thickness direction of the absorbent core is not consolidated.
<16> The absorbent article according to any one of <1> to <14>, wherein the absorbent core has a density of preferably 0.03 to 0.2 g/cm³, more preferably 0.06 to 0.15 g/cm³.
<17> The absorbent article according to any one of <1> to <16>, wherein
   the absorbent article is folded in two or three in a longitudinal direction,
   a plurality of pierced or non-pierced slits, each of which extends in a longitudinal direction of the article, are formed on the absorbent core, and
   the slits are not connected consecutively to each other in at least a portion under a crotch, and have a wide portion having a wide slit width at a position different from the position at which the article is folded in two or three.
<18> The absorbent article according to any one of <1> to <17>, wherein the slit is a non-pierced slit which opens on only a backsheet side.
<19> The absorbent article according to <17> or <18>, wherein the absorbent article is folded in two in the longitudinal direction, and
   the wide portion is formed on a front portion side from the position at which the article is folded in two.
<20> The absorbent article according to <19>, wherein the wide portion is further formed on a rear portion side from the portion at which the article is folded in two.
<21> The absorbent article according to any one of <17> to <20>, wherein each of the slits has a plurality of wide portions on the front portion from the position at which the article is folded in two.
<22> The absorbent article according to any one of <1> to <21>, wherein a space is formed inside the projections of the topsheet.
<23> The absorbent article according to any one of <1> to <22>, wherein the topsheet has an upper layer facing the skin side of a wearer and a lower layer arranged on the absorbent member side; the upper layer and the lower layer are partly joined to each other on the bottom of each of a large number of the depressions; the bottom of each depression is a junction between the upper layer and the lower layer; and the upper layer protrudes toward the wearer skin side in parts other than the bottom (junction) of the depression to form the projection.
<24> The absorbent article according to any one of <1> to <23>, wherein the absorbent article is a disposable diaper.

### Examples

The present invention will be specifically explained by means of Examples below, but the present invention is not limited thereto.

### [Example 1]

An open type disposable diaper having a basic configuration, which was the same as that of the diaper 1 shown in FIG. 1 and FIG 2, was produced, and it was used as a sample of Example 1. As a topsheet, a sheet having the almost same configuration as that of the uneven topsheet having the two-layer configuration shown in FIG. 3; as each of an upper layer and a lower layer of the topsheet, a liquid-permeable and air-through non-woven fabric having a basis weight of 18 g/m² was used; and as the upper layer, the air-through non-woven fabric on which unevenness was provided by emboss processing was used. The basis weight of the upper layer was 23.5 g/m² after the unevenness was provided. As a backsheet, a liquid-impermeable and moisture-permeable polyethylene resin film (including calcium carbonate) having a basis weight of 20 g/m² was used. As an absorbent core, an absorbent core including a uniform mixture of 200 g/m² of a fluff pulp and 186 g/m² of an absorbent polymer, having a total basis weight of 386 g/m², was used as a fiber assembly in which a particulate absorbent polymer was held. The absorbent core had a full length of 360 mm in the longitudinal direction, and a full length (the maximum length) of 110 mm in the width direction. Slits were formed in the manner as described above in which a predetermined mold was used, and materials forming the absorbent core were not intentionally accumulated in predetermined portions during the production of the absorbent core. As a core wrap sheet, a core wrap sheet A1, which was produced in a method described below, was used.

### <Production Method of Core Wrap Sheet A1>

NBKP [prepared by mixing two kinds of pulps (NBKPs), a trade name "Cariboo" (grown in North America, manufactured by Cariboo Pulp and Paper Company) and a trade name "ARAUCO" (grown in South America, manufactured by ARAUCO) in a mas ratio of the two pulps contained of 5/5] was uniformly dispersed in water to prepare slurry having a fiber concentration of 2% by mass, and this slurry was adjusted to a freeness of NBKP of 500 ml passing through a beating machine. Further, while the slurry was diluted, 0.78% by mass, based on the mass of the all dry fibers in the slurry, of PAE (a trade name "WS 4030" manufactured by Seiko PMC Corporation) was thrown into the slurry as a wet paper strong agent, and then 0.2% by mass, based on the mass of the all dry fibers in the slurry, of a sodium salt of CMC (a trade name "Cellogen WS-C" manufactured by Dai-Ichi Kogyo Seiyaku Co., Ltd) was thrown thereinto as a dry paper strong agent, and the mixture was thoroughly stirred so that each component was uniform, thereby preparing slurry having a solid concentration of 0.1% by mass. The thus obtained slurry was sprayed on a wire netting wire for paper-making, which had a wire aperture of 90 µm (166 mesh), to form a paper layer on the wire netting wire for paper-making. After the paper layer was dehydrated using a suction box at a speed of 6 ml/(cm²·sec), the paper layer was dried with a dryer, and while the paper layer was peeled off from the dry surface with a doctor blade, crepe was provided by a velocity ratio of the dryer and a take-up speed. The thus obtained tissue paper (crepe paper) was used as a core wrap sheet A1.

### [Example 2]

An open type disposable diaper was produced in the same manner as in Example 1 except that a core wrap sheet A2 was used instead of the core wrap sheet A1, which was used as a sample of Example 2. The core wrap sheet A2 was produced in the same manner as in <Production Method of Core Wrap Sheet Al> except that the freeness of NBKP was adjusted to 550 ml.

### [Comparative Example 1]

An open type disposable diaper was produced in the same manner as in Example 1 except that a core wrap sheet B, which had been produced in a method described below, was used instead of the core wrap sheet A1, which was used as a sample of Comparative Example 1.

### <Production Method of Core Wrap Sheet B>

NBKP (a trade name "Cariboo" manufactured by Cariboo Pulp and Paper Company, grown in North America, a fiber roughness: 0.15 mg/m, and average fiber length: 2.44 mm) was uniformly dispersed in water to prepare slurry having a fiber concentration of 2% by mass, and a freeness of NBKP was adjusted to 650 ml by passing through a beating machine. Further, while this slurry was diluted, 0.78% by mass, based on the mass of the all dry fibers in the slurry, of PAE (a trade name "WS 4030" manufactured by Seiko PMC Corporation) was thrown into the slurry as a wet paper strong agent, and the mixture was thoroughly stirred so that each component was uniform, thereby preparing slurry having a solid concentration of 0.1% by mass. The thus obtained slurry was sprayed on a wire netting wire for paper-making, which had a wire aperture of 90 µm (166 mesh), to form a paper layer on the wire netting wire for paper-making. After the paper layer was dehydrated using a suction box at a speed of 6 ml/ (cm²·sec), the paper layer was dried with a dryer, and while the paper layer was peeled off from the dry surface with a doctor blade, crepe was provided by a velocity ratio of the dryer and a take-up speed. The thus obtained tissue paper (crepe paper) was used as a core wrap sheet B.

### [Comparative Example 2]

An open type disposable diaper was produced in the same manner as in Example 1 except that slits were not formed in the absorbent core, which was used as a sample of Comparative Example 2.

### [Comparative Example 3]

An open type disposable diaper was produced in the same manner as in Example 2 except that the core wrap sheet B was used instead of the core wrap sheet A1, which was used as a sample of Comparative Example 3.

### [Comparative Example 4]

An open type disposable diaper was produced in the same manner as in Example 1 except that a flat air-through non-woven fabric having no unevenness (basis weight: 25 g/m²) was used as the topsheet instead of the uneven topsheet having two-layer configuration, which was used as a sample of Comparative Example 4.

### [Evaluation]

With respect to each sample (disposable diaper) from Examples and Comparative Examples, a wet back amount, a urine absorption time, and a loose stool absorbency were evaluated in accordance with methods described below. Those results are shown in Table 1 below.

### <Wet Back Amount and Urine Absorption Time>

The disposable diaper was expanded into a planar shape, and the topsheet was turned up and fixed on a horizontal plane. Keeping this state, an acrylic plate provided with a cylindrical injection port was put on the topsheet of the diaper, a weight was put on the acrylic plate, and a load of 2 kPa (20 gf/cm²) was applied to the absorbent member part. The injection port provided on the acrylic plate had a cylindrical shape (height: 53 mm) having an inside diameter of 36 mm. On the acrylic plate was formed a through hole having an inside diameter of 36mm, whose central axis was agreed with a central axis in the width direction at a one third portion in the longitudinal direction, and which connects the inside of the cylindrical injection port to a surface facing to the topsheet of the acrylic plate. The acrylic plate was arranged so that the central axis of the cylindrical injection port on the acrylic plate was set at a position 123 mm from a tip end of a front side portion in the longitudinal direction of the core wrap sheet, which covers the absorbent core of the diaper. 30 g of artificial urine was injected, it was absorbed, it was allowed to stand for 10 minutes, 30 g of artificial urine was further injected, and it was absorbed. The injection procedure of the artificial urine was repeated three times, whereby the total amount of 90 g of the artificial urine was absorbed in the diaper. After that, 20 4A filter papers (10 cm × 10 cm), manufactured by Toyo Roshi Kaisha, Ltd. was put on an absorption portion of the artificial urine of the diaper, to which a load was added, which was allowed to stand for 2 minutes, whereby the artificial urine, which had been absorbed in the diaper, was absorbed in the filter papers. The load of 3.5 kg was applied to an area of 10 cm × 10 cm. After 2 minutes, the load was removed, and a weight amount of the filter papers absorbing the artificial urine was measured. The weight amount of the filter papers before the absorption of the artificial urine was subtracted from the measured weight amount, and the obtained value was defined as a wet back amount. Also, in the measurement of the wet back amount, an injection time of the artificial urine injection at the third time (a time from a starting time of the injection to a time at which the total amount was absorbed in the diaper) was defined as the urine absorption time. The smaller the wet back amount or the shorter the urine absorption time, the more excellent the absorbency of the excrement liquid and the higher the evaluation.

The artificial urine has the following composition: urea: 1.94% by mass; sodium chloride: 0.7954% by mass; magnesium sulfate (heptahydrate): 0.11058% by mass; calcium chloride (dihydrate): 0.06208% by mass; potassium sulfate: 0.19788% by mass; polyoxyethylene lauryl ether: 0.0035% by mass; and deionized water (residual quantity).

### <Loose Stool Absorbency>

The disposable diaper was expanded into a planar shape, and the topsheet was turned up and fixed on a horizontal plane. Keeping this state, 5 g of spurious loose stool, which was a model of high viscosity liquid, was injected from the topsheet side into a center part of the absorbent member at a constant speed (6 seconds) at once using a syringe, which was allowed to stand for 5 minutes under a load of 3.5 kPa. At this time, an OHP film (15 × 15 cm) was put between the weight for applying the load and the disposable diaper. After that, an amount of the spurious loose stool adhering to the OHP film was measured, the measured value was defined as an adhesion amount to skin, and a diffusion area of the loose stool on the topsheet was measured. Also, the topsheet was peeled off, and a weight amount of the spurious loose stool which was absorbed in the absorbent member was measured, which was defined as an absorption amount of loose stool. The smaller the adhesion amount to skin or the larger the absorption amount of loose stool, the more excellent the loose stool absorbency and the higher the evaluation. The spurious loose stool had components of 110 g of bentonite, 45 g of glycerol, 495 g of deionized water, and 0.35 g of Emulgen 130 K (Kao Chemicals), and had a viscosity of 300 mPa·s (at 23°C, a vibration type viscometer, VIBRO VISCOMETER CJV5000 manufactured by A & D Company, Limited).

**[Table 1]**

| | | | Example | | Comparative Example | | | |
|---|---|---|---|---|---|---|---|---|
| | | | 1 | 2 | 1 | 2 | 3 | 4 |
| Structure of Absorbent article | Uneven Shape of Topsheet | | Presence | Presence | Presence | Presence | Presence | Absence |
| | Core Wrap Sheet | Liquid Permeation Time of Low Viscosity Liquid (second) | 0.9 | 2.2 | 5.0 | 0.9 | 5.0 | 0.9 |
| | | Liquid Permeation Time of High Viscosity Liquid (second) | 175 | 290 | 960 | 175 | 960 | 175 |
| | | Basis Amount(g/m²) | 13.5 | 13.5 | 16 | 13.5 | 16 | 13.5 |
| | Slit of Absorbent core | | Presence | Presence | Presence | Absence | Absence | Presence |
| Property Evaluation | Wet Back Amount(g) | | 0.38 | 0.44 | 0.58 | 0.46 | 0.55 | 0.82 |
| | Urine Absorption Time (second) | | 67.5 | 69.7 | 70.3 | 94.5 | 81.5 | 95.5 |
| | Loose Stool Absorbency | Adhesion Amount to Skin(mg) | 90 | 75 | 190 | 115 | 185 | 120 |
| | | Absorption Amount of Loose Stool(g) | 3.71 | 3.38 | 2.64 | 3.56 | 2.84 | 4.57 |
| | | Diffusion Area (cm²) | 35.9 | 33.3 | 42.0 | 38.4 | 45.6 | 40.3 |

As apparent from the results shown in Table 1, the disposable diapers of Examples 1 and 2 have smaller wet back amounts and shorter urine absorption times with respect to the urine, and smaller adhesion amounts to skin and larger absorption amounts of loose stool and smaller diffusion area with respect to the loose stool, compared with the disposable diaper of each Comparative Example. It is accordingly found that the disposable diapers of Examples 1 and 2 are absorbent articles having the excellent absorbency to the excrement liquid such as urine or loose stool, in which it is difficult to adhere the excrement liquid to the skin of the wearer. In Comparative Example 1, the liquid permeation time of a low viscosity liquid of the core wrap sheet was more than 2 seconds, in Comparative Examples 2 and 3, slits are not formed in the absorbent core, and in Comparative Example 4, the topsheet does not have projections protruding toward the skin side of a wearer. In Comparative Examples, these facts mainly causes the results of absorbency to the excrement liquid inferior to those in each Example.

### [Example 11]

An open type disposable diaper having a basic configuration, which was the same as that of the diaper 101 shown in FIG. 7 and FIG. 8, was produced, and it was used as a sample of Example 11. As a topsheet, uneven topsheet having a two-layer configuration was used which has an upper layer facing skin side of a wearer and a lower layer arranged on an absorbent member side, and has a large number of projections protruding toward the skin side of the wearer and a large number of depression formed on a skin-facing surface of the topsheet. As each of the upper layer and the lower layer on the uneven topsheet having the two-layer configuration, a liquid-permeable air-through non-woven fabric having a basis weight of 18 g/m² was used, and as the upper layer, the air-through non-woven fabric on which unevenness was provided by emboss processing was used. The basis weight of the upper layer was 23.5 g/m² after the unevenness was provided. In the uneven topsheet having the two-layer configuration, the upper layer having the unevenness and the lower layer having no unevenness were partly joined to each other at bottoms of large number of the depressions, and the bottom of each depression was a junction between the upper layer and the lower layer. In addition, portions other than bottoms of the depression (junctions) of the upper layer protruded toward the skin side of the wearer to form projections, and a space was formed inside the projection. As a backsheet, a liquid-impermeable and moisture-permeable polyethylene resin film (including calcium carbonate) having a basis weight of 20 g/m² was used. As an absorbent core, an absorbent core including a uniform mixture of 200 g/m² of a fluff pulp and 186 g/m² of an absorbent polymer, having a total basis weight of 386 g/m², was used as a fiber assembly in which a particulate absorbent polymer was held. The absorbent core had a full length of 360 mm in the longitudinal direction, and a full length (the maximum length) of 110 mm in the width direction. Slits were formed in the manner as described above in which a predetermined mold was used, and materials forming the absorbent core were not intentionally accumulated in predetermined portions during the production of the absorbent core. The slit had, as shown in FIG. 2, a shape of (isosceles) trapezoid in a section view in the width direction, and the width was gradually increased from the skin-facing surface side to the non-skin-facing surface side in the whole area of the slit. As a core wrap sheet, a liquid-permeable cardboard having a basis weight of 13.5 g/m² was used.

### [Reference Example 11]

An open type disposable diaper was produced in the same manner as in Example 11 except that slits were not formed in the absorbent core, which was used as a sample of Reference Example 11.

### [Reference Example 12]

An absorbent core having no slit was produced in the same manner as in Reference Example 11, and slits were formed by compressing predetermined portions of the absorbent core in the thickness direction by known emboss processing. A portion facing the opening of the slit (the portion facing the opening), in the thus formed absorbent core, was consolidated, and thus it had a density higher than that of the peripheral part thereof. The slit had a rectangular shape in a section view in the width direction, as shown in FIG. 8, and the width was not changed in the thickness direction of the absorbent core in the whole area of the slit. An open type disposable diaper was produced in the same manner as in Example 11 except for the points described above, which was used as a sample of Reference Example 12.

### [Reference Example 13]

An open type disposable diaper was produced in the same manner as in Example 11 except that pierced slits which pierced the absorbent core in the thickness direction were used as the slits on the absorbent core, and the absorbent core having no portion facing the opening was used, which was used as a sample of Reference Example 13.

### [Evaluation]

With respect to each sample (disposable diaper) from Example and Reference Examples, a bending rigidity, a wet back amount, and a urine absorption time were evaluated as above and in accordance with a method described below. Those results are shown in Table 2 below.

### <Bending Rigidity>

Using a handle-o-meter, a bending rigidity of a sample for measurement was measured. The smaller the value measured by the handle-o-meter, the sample for measurement is more easily bent (bending deformation occurs more easily, the slit described above serving as a line of bending), the diaper is softer and more easily worn, and the fit is better. The measurement method of the bending rigidity using the handle-o-meter is as follows: A sample for measurement having the almost same width as the absorbent member was produced by removing the three-dimensional gather from the diaper sample, and the sample for measurement was arranged on a support of the handle-o-meter having a channel with a width of 20 mm so that the longitudinal direction of the sample for measurement was perpendicular to the channel. A center of the sample for measurement (absorbent member) was pushed with a blade having a thickness of 2 mm, whereby a force necessary for bending the sample for measurement was measured. An average value of three values obtained at the center of the sample for measurement was defined as a measurement value. As the handle-o-meter, a feeling tester (handle-o-meter method), HOM-3 Type, manufactured by Daiei Kagaku Seiki MFG. Co., Ltd, may be used.

**[Table 2]**

| | | Example | Reference Example | | |
|---|---|---|---|---|---|
| | | 11 | 11 | 12 | 13 |
| Slit of Absorbent core | Kind of Slit | non-pierced | no slit | non-pierced | pierced |
| | Consolidating of Portion Facing Opening | none | - | done | - |
| | Cross-Sectional Shape in Width Direction | trapezoidal shape | - | rectangular shape | trapezoidal shape |
| Property Evaluation | Bending Rigidity (Handle-O-Meter)(g) | 84 | 89 | 99 | 79 |
| | Wet Back Amount(g) | 0.45 | 0.54 | 0.51 | 0.79 |
| | Urine Absorption Time (second) | 67 | 81 | 87 | 75 |

As apparent from the results shown in Table 2, the disposable diaper of Example 11 had the smaller wet back amount, the shorter urine absorption time, and the lower bending rigidity, and was softer than those of the disposable diaper of each of Reference Examples 11 to 13, and it was found therefore that the diaper is an absorbent article having the good fit. With respect to the disposable diaper of Reference Example 11, largely because slits were not formed on the absorbent core, the liquid-permeating and diffusing function to the excrement liquid is poor, thus resulting in the large wet back amount and long urine absorption time. In addition, because the bending deformation cannot be caused, the slit serving as the line of bending, the bending rigidity is high, and the softness and the fit are poor. With respect to the disposable diaper of Reference Example 12, although the slits are formed on the absorbent core, the cross-sectional shape of the slit in the width direction is the rectangular shape, and the width does not change in the thickness direction of the absorbent core, the bending rigidity is high, and the softness and the fit are poor. In addition, in the disposable diaper of Reference Example 12, because the portion facing the opening is consolidated due to the formation of the slits by the emboss processing, it is difficult to cause the bending deformation, the slit serving as the line of bending, and because it is easy to delay the liquid diffusion at the portion facing the opening, which has a density higher than that of the peripheral part, the wet back amount is large and the urine absorption time is long. With respect to the disposable diaper of Reference Example 13, because the slit formed on the absorbent core is the pierced slit, the urine absorption time is short (the urine absorption speed is fast), but the wet back amount is large, and thus it cannot be said that the absorbency to excrement liquid is good. From the above, it is found that it is effective for improving both of the fit and the leakage prevention property of the diaper to form non-pierced slits on the absorbent core, to adjust the cross-sectional shape of the slit in the width direction to the trapezoidal shape, and not to consolidate the portion facing the opening of the slit.

### Industrial Applicability

According to the present invention, the absorbent article having good absorbency to the excrement liquid such as urine or loose stool, and the reduced adhesion of the excrement liquid to the skin of the wearer is provided.

The disposable diaper of the second embodiment has good fit to the body of the wearer, and reduced leakage of the excrement liquid.

In addition, according to the disposable diapers of third and fourth embodiments, even if creases are generated, the good effect of improving the fit to the crotch, which is obtained by the slits in the longitudinal direction, is exhibited, and thus good fit and leakage prevention property are obtained.

## Claims

1. An absorbent article comprising an absorbent member (4) and a topsheet (2, 102) which is arranged on a skin-facing surface side of the absorbent member (4), the absorbent member (4) including an absorbent core (40, 140) and a core wrap sheet (41, 141) which covers at least a skin-facing surface of the absorbent core (40, 140), wherein
the topsheet (2, 102) has a large number of projections (25) which protrude toward the skin of a wearer,
the absorbent core (40, 140) has a slit (5, 105) extending in a predetermined direction and having an opening (50, 150) on a non-skin-facing surface (40b, 140b) side of the absorbent core (40, 140), wherein the slit (5, 105) does not pierce the absorbent core (40, 140) in a thickness direction and wherein a forming material of the absorbent core (40, 140) is accumulated between the slit (5, 105) and the portion of the core wrap sheet (41, 141) which covers the skin-facing surface of the absorbent core (40, 140), and wherein a portion of the absorbent core (40, 140) which is adjacent to the slit (5, 105) in a thickness direction of the absorbent core (40, 140) is not consolidated, and
a portion of the core wrap sheet (41, 141) which covers the skin-facing surface of the absorbent core (40, 140) has a liquid permeation time of a low viscosity liquid, as measured in the following method, of 2.5 seconds or less,
<Measurement Method of Liquid Permeation Time of Low Viscosity Liquid>
wherein the liquid permeation time of a low viscosity liquid is measured using two cylinders (91, 92) having an inside diameter of 35 mm, whose upper and lower ends are open, are arranged up and down bringing axis of the both cylinders in line, a sheet to be measured (core wrap sheet) is put between the upper and bottom cylinders, and, while that condition is kept, 40 g ± 1 g of physiological saline is supplied into the upper cylinder, the 40 g ± 1 g of physiological saline being poured into the upper cylinder at once without pausing from a container including the physiological saline, thereby supplying the saline into the upper cylinder, wherein the physiological saline supplied permeates through the sheet to be measured or is absorbed into the sheet to be measured, whereby it disappears from the upper cylinder, and wherein a time until a liquid surface of the physiological saline reaches a position of a surface of the sheet to be measured from the supply starting time of the physiological saline is measured, and the time is defined as a liquid permeation time.

2. The absorbent article according to claim 1, wherein the portion of the core wrap sheet (41, 141) which covers the skin-facing surface of the absorbent core (40, 140) has a liquid permeation time of a low viscosity liquid, as measured in the method described in claim 1, of 0.5 to 2.5 seconds.

3. The absorbent article according to claim 1 or 2, wherein the slit (5, 105) has a portion in which a width of the slit (5, 105) increases gradually from the skin-facing surface (40a, 140a) side toward the non-skin-facing surface (40b, 140b) side in the absorbent core.

4. The absorbent article according to any one of claims 1 to 3, wherein the slit (5, 105) is a continuous straight line slit, a plurality of lines being formed at predetermined intervals in a lateral direction of the absorbent article.

5. The absorbent article according to any one of claims 1 to 4, wherein
the absorbent article is folded in two or three in a longitudinal direction,
a plurality of pierced or non-pierced slits (5, 105), each of which extends in a longitudinal direction of the article, are formed on the absorbent core (40, 140), and
the slits (5, 105) are not connected consecutively to each other in at least a portion under a crotch, and have a wide portion having a wide slit width at a position different from the position at which the article is folded in two or three.

6. The absorbent article according to any one of claims 1 to 5, wherein the slit (5, 105) opens on only a backsheet side.

7. The absorbent article according to claim 5 or 6, wherein the absorbent article is folded in two in the longitudinal direction, and
the wide portion is formed on a front portion side from the position at which the article is folded in two.

8. The absorbent article according to claim 7, wherein the wide portion is further formed on a rear portion side from the portion at which the article is folded in two.

9. The absorbent article according to any one of claims 5 to 8, wherein each of the slits (5, 105) has a plurality of wide portions on the front portion side from the position at which the article is folded in two.

10. The absorbent article according to any one of claims 1 to 9, wherein a space is formed inside the projections (25, 125) of the topsheet (4, 104).

## Patentansprüche

1. Absorbierender Artikel, aufweisend ein absorbierendes Element (4) und eine Oberschicht (2, 102), die an der Seite einer hautzugewandten Oberfläche des absorbierenden Elements (4) angeordnet ist, wobei das absorbierende Element (4) einen absorbierenden Kern (40, 140) und eine Kernhüllschicht (41, 141) aufweist, die mindestens eine hautzugewandte Oberfläche des absorbierenden Kerns (40, 140) bedeckt, wobei
die Oberschicht (2, 102) eine große Anzahl von Vorsprüngen (25) hat, die zur Haut eines Trägers hin vorspringen,
der absorbierende Kern (40, 140) einen Schlitz (5, 105) hat, der sich in eine vorgegebene Richtung erstreckt und eine Öffnung (50, 150) auf der Seite einer nichthautzugewandten Oberfläche (40b, 140b) des absorbierenden Kerns (40, 140) hat, wobei der Schlitz (5, 105) den absorbierenden Kern (40, 140) in einer Dickenrichtung nicht durchdringt, und wobei ein Formmaterial des absorbierenden Kerns (40, 140) zwischen dem Schlitz (5, 105) und dem Teil der Kernhüllschicht (41, 141), der die hautzugewandte Oberfläche des absorbierenden Kerns (40, 140) bedeckt, angehäuft ist, und wobei ein Teil des absorbierenden Kerns (40, 140), der an den Schlitz (5, 105) in einer Dickenrichtung des absorbierenden Kerns (40, 140) angrenzt, nicht verdichtet ist, und
ein Teil der Kernhüllschicht (41, 141), der die hautzugewandte Oberfläche des absorbierenden Kerns (40, 140) bedeckt, eine mit dem folgenden Verfahren gemessene Flüssigkeitsdurchlasszeit einer niederviskosen Flüssigkeit von 2,5 Sekunden oder weniger hat.
<Verfahren zum Messen einer Flüssigkeitsdurchlasszeit einer niederviskosen Flüssigkeit>
wobei die Flüssigkeitsdurchlasszeit einer niederviskosen Flüssigkeit gemessen wird unter Verwendung von zwei Zylindern (91, 92) eines Innendurchmessers von 35mm, deren obere und untere Enden offen sind, wobei die beiden Zylinder mit in einer Linie ausgerichteten Achsen übereinander angeordnet werden und eine zu messende Schicht (Kernhüllschicht) zwischen dem oberen und unteren Zylinder angeordnet wird und unter Beibehaltung dieses Zustands 40g±1g physiologische Kochsalzlösung in den oberen Zylinder gegeben wird, wobei die 40g±1g physiologische Kochsalzlösung aus einem Behälter, der die physiologische Kochsalzlösung enthält, auf ein Mal ohne Pause in den oberen Zylinder geschüttet wird, um dadurch die Kochsalzlösung in den oberen Zylinder zu geben, wobei die zugeführte Kochsalzlösung die zu messende Schicht durchdringt oder in der zu messenden Schicht absorbiert wird, wodurch sie aus dem oberen Zylinder verschwindet, und wobei eine Zeit, bis ein Flüssigkeitsspiegel der physiologischen Kochsalzlösung eine Position einer Oberfläche der zu messenden Schicht erreicht hat, ab dem Startzeitpunkt der Zuführung der physiologischen Kochsalzlösung gemessen wird, und die Zeit als Flüssigkeitsdurchlasszeit definiert wird.

2. Absorbierender Artikel nach Anspruch 1, wobei der Teil der Kernhüllschicht (41, 141), der die hautzugewandte Oberfläche des absorbierenden Kerns (40, 140) bedeckt, eine Flüssigkeitsdurchlasszeit einer niederviskosen Flüssigkeit, wie in dem in Anspruch 1 beschriebenen Verfahren gemessen, von 0,5 bis 2,5 Sekunden hat.

3. Absorbierender Artikel nach Anspruch 1 oder 2, wobei der Schlitz (5, 105) einen Abschnitt hat, in welchem eine Breite des Schlitzes (5, 105) von der Seite der hautzugewandten Oberfläche (40a, 140a) zur Seite der nichthautzugewandten Oberfläche (40b, 140b) in dem absorbierenden Kern allmählich zunimmt.

4. Absorbierender Artikel nach einem der Ansprüche 1 bis 3, wobei der Schlitz (5, 105) ein kontinuierlicher geradliniger Schlitz ist und mehrere Linien in einer lateralen Richtung des absorbierenden Artikels in vorgegebenen Abständen gebildet sind.

5. Absorbierender Artikel nach einem der Ansprüche 1 bis 4, wobei
der absorbierende Artikel in einer Längsrichtung ein oder zwei Mal gefaltet ist,
mehrere durchdringende oder nichtdurchdringende Schlitze (5, 105), die sich jeweils in eine Längsrichtung des Artikels erstrecken, an dem absorbierenden Kern (40, 140) gebildet sind, und
die Schlitze (5, 105) in mindestens einem Teil unter dem Schritt nicht fortlaufend miteinander verbunden sind und an einer anderen Position als an derjenigen, wo der Artikel ein oder zwei Mal gefaltet ist, einen weiten Abschnitt mit einer weiten Schlitzbreite haben.

6. Absorbierender Artikel nach einem der Ansprüche 1 bis 5, wobei der Schlitz (5, 105) nur auf der Seite einer Rückschicht offen ist.

7. Absorbierender Artikel nach Anspruch 5 oder 6, wobei der absorbierende Artikel in der Längsrichtung ein Mal gefaltet ist und der weite Abschnitt auf der Seite eines vorderen Abschnitts von der Position aus, an der der Artikel ein Mal gefaltet ist, gebildet ist.

8. Absorbierender Artikel nach Anspruch 7, wobei der weite Abschnitt außerdem auf der Seite eines hinteren Abschnitts von der Position aus, an der der Artikel ein Mal gefaltet ist, gebildet ist.

9. Absorbierender Artikel nach einem der Ansprüche 5 bis 8, wobei jeder der Schlitze (5, 105) mehrere weite Abschnitte auf der Seite des vorderen Abschnitts von der Position aus, an der der Artikel ein Mal gefaltet ist, hat.

10. Absorbierender Artikel nach einem der Ansprüche 1 bis 9, wobei ein Raum im Innern der Vorsprünge (25, 125) der Oberschicht (4, 104) gebildet ist.

## Revendications

1. Article absorbant comprenant un élément absorbant (4) et une feuille supérieure (2, 102) qui est disposée sur un côté de surface tournée vers la peau de l'élément absorbant (4), l'élément absorbant (4) comprenant un coeur absorbant (40, 140) et une feuille enveloppant le coeur (41, 141) qui couvre au moins une surface tournée vers la peau du corps absorbant (40, 140), dans lequel
la feuille supérieure (2, 102) a un grand nombre de protubérances (25) qui font saillie vers la peau d'un utilisateur,
le coeur absorbant (40, 140) a une fente (5, 105) s'étendant dans une direction prédéterminée et ayant une ouverture (50, 150) sur un côté de surface non tournée vers la peau (40b, 140b) du corps absorbant (40, 140), où la fente (5, 105) ne perce pas le coeur absorbant (40, 140) dans la direction de l'épaisseur, et un matériau formant le coeur absorbant (40, 140) est accumulé entre la fente (5, 105) et la partie de la feuille enveloppant le coeur (41, 141) qui couvre la surface tournée vers la peau du coeur absorbant (40, 140), et une partie du coeur absorbant (40, 140) qui est adjacente à la fente (5, 105) dans la direction de l'épaisseur du coeur absorbant (40, 140) n'est pas consolidée, et
une partie de la feuille enveloppant le coeur (41,141) qui couvre la surface tournée vers la peau du coeur absorbant (40, 140) a un temps de perméation de liquide pour un liquide faiblement visqueux, tel que mesuré dans le procédé qui suit, de 2,5 secondes ou moins,
<Procédé de mesure du temps de perméation de liquide pour un liquide faiblement visqueux>
où le temps de perméation de liquide d'un liquide faiblement visqueux est mesuré par utilisation de deux cylindres (91, 92) ayant un diamètre intérieur de 35 mm, dont les extrémités supérieure et inférieure sont ouvertes et agencées en haut et en bas en alignant l'axe des deux cylindres, une feuille devant être mesurée (feuille enveloppant le coeur) est placée entre les cylindres supérieur et inférieur et, cependant que cette condition est maintenue, 40 g ± 1 g de solution salée physiologique sont introduits dans le cylindre supérieur, les 40 g ± 1 g de solution salée physiologique étant versés dans le cylindre supérieur en une seule fois sans pause à partir d'un récipient contenant la solution salée physiologique, ce qui délivre ainsi la solution salée dans le cylindre supérieur, où la solution salée physiologique délivrée traverse par perméation la feuille devant être mesurée ou est absorbée dans la feuille devant être mesurée, si bien qu'elle disparaît du cylindre supérieur, et où le temps pour qu'une surface liquide de la solution salée physiologique atteigne une position d'une surface de la feuille devant être mesurée à partir du temps de début de délivrance de la solution salée physiologique est mesuré, et le temps est défini comme étant le temps de perméation de liquide.

2. Article absorbant selon la revendication 1, dans lequel la partie de la feuille enveloppant le coeur (41, 141) qui couvre la surface tournée vers la peau du coeur absorbant (40, 140) a un temps de perméation de liquide pour un liquide faiblement visqueux, tel que mesuré dans le procédé décrit dans la revendication 1, de 0,5 à 2,5 secondes.

3. Article absorbant selon la revendication 1 ou 2, dans lequel la fente (5, 105) a une partie dans laquelle la largeur de la fente (5, 105) augmente progressivement à partir du côté de surface tournée vers la peau (40a, 140a) en direction du côté de surface non tournée vers la peau (40b, 140b) dans le coeur absorbant.

4. Article absorbant selon l'une quelconque des revendications 1 à 3, dans lequel la fente (5, 105) est une fente formant une ligne droite continue, une pluralité de lignes étant formées à intervalles prédéterminés dans la direction latérale de l'article absorbant.

5. Article absorbant selon l'une quelconque des revendications 1 à 4, dans lequel
l'article absorbant est plié en deux ou trois dans la direction longitudinale,
une pluralité de fentes percées ou non percées (5, 105), qui s'étendent chacune dans la direction longitudinale de l'article, sont formées sur le coeur absorbant (40, 140), et
les fentes (5, 105) ne sont pas connectées consécutivement les unes aux autres dans au moins une partie sous l'entrejambe, et ont une partie large ayant une grande largeur de fente en une position différente de la position à laquelle l'article est plié en deux ou trois.

6. Article absorbant selon l'une quelconque des revendications 1 à 5, dans lequel la fente (5, 105) s'ouvre seulement sur un côté de feuille d'envers.

7. Article absorbant selon la revendication 5 ou 6, lequel article absorbant est plié en deux dans la direction longitudinale, et dans lequel la partie large est formée sur un côté de partie avant par rapport à la position à laquelle l'article est plié en deux.

8. Article absorbant selon la revendication 7, dans lequel la partie large est en outre formée sur un côté de partie arrière par rapport à la partie au niveau de laquelle l'article est plié en deux.

9. Article absorbant selon l'une quelconque des revendications 5 à 8, dans lequel chacune des fentes (5, 105) a une pluralité de parties larges sur le côté de partie avant par rapport à la position à laquelle l'article est plié en deux.

10. Article absorbant selon l'une quelconque des revendications 1 à 9, dans lequel un espace est formé à l'intérieur des protubérances (25, 125) de la feuille supérieure (4, 104).
